# EUROPÄISCHE PATENTSCHRIFT

(11) **EP 0 767 913 B1**
(45) Veröffentlichungstag und Bekanntmachung des Hinweises auf die Patenterteilung: **09.10.2002**
(21) Anmeldenummer: 95924919.4
(22) Anmeldetag: 23.06.1995
(51) Int. Cl.: G01N 33/542, G01N 33/543, G01N 33/58, G01N 33/577, C12Q 1/68

(54) **VERFAHREN ZUR DURCHFÜHRUNG EINES IMMUNOASSAYS IN EINEM MEHRPHASENSYSTEM**
METHOD OF CARRYING OUT AN IMMUNO-ASSAY IN A MULTI-PHASE SYSTEM
PROCEDE DE MISE EN OEUVRE D'UN DOSAGE IMMUNOLOGIQUE DANS UN SYSTEME MULTIPHASE

(30) Priorität: 24.06.1994 DE 4421907
(43) Veröffentlichungstag der Anmeldung: 16.04.1997
(73) Patentinhaber: Dade Behring Marburg GmbH, 35001 Marburg (DE)
(72) Erfinder: NEUENHOFER, Stephan, D-35037 Marburg (DE); KÄSMARKER, Reinhard, D-65824 Schwalbach/Ts. (DE)
(86) Internationale Anmeldenummer: EP9502447
(87) Internationale Veröffentlichungsnummer: WO96000393

(56) Entgegenhaltungen:
- EP-A- 0 017 908
- EP-A- 0 272 691
- EP-A- 0 328 106
- EP-A- 0 476 545
- CHEMICAL ABSTRACTS, vol. 91, no. 9, 27.August 1979 Columbus, Ohio, US; abstract no. 71265, R. D. NARGESSI ET AL. 'Use of antibodies against the label in non-separation non-isotopic immunoassay: 'indirect quenching' fluoroimmunoassay of proteins.' Seite 305; Spalte 1; & J. IMMUNOL. METHODS, Bd. 26, Nr. 4, 1979 Seiten 307-313,

## Beschreibung

Die vorliegende Erfindung betrifft ein Verfahren zur Durchführung eines Immunoassays in einem Mehrphasensystem.

Immunologische Nachweisverfahren haben in der in vitro Diagnostik eine hohe Bedeutung erlangt. Ursache hierfür ist, daß sie hochspezifisch und äußerst empfindlich sind. Zudem zeichnen sich diese Assays durch eine einfache Handhabung aus. Die Nachweisverfahren beruhen auf der immunologischen Wechselwirkung zwischen dem nachzuweisenden Analyten und seinem Bindungspartner bzw. -partnern.

Im Falle der Sandwich-Assays wird der Analyt von zwei verschiedenen Antikörpern sandwichartig gebunden. Einer der beiden Antikörper trägt eine Markierung (Label, Marker), wodurch seine Konzentration bestimmt werden kann.

Im Falle kleiner Analyte scheidet das Sandwich-Verfahren aus, da z. B. aus sterischen Gründen nicht gleichzeitig zwei verschiedene Antikörper am Analyten binden können. Hier finden in der Regel die kompetitiven Assays Anwendung. Dabei konkurrieren z. B. ein Analyt und ein synthetisches Derivat des Analyten um die Bindungsstellen des Antikörpers. Markiert wird in der Regel entweder das Analytderivat (klassisch kompetitives Verfahren) oder der Antikörper (z.B. SPALT: solid phase antigen luminescence technique). Die markierte Komponente wird als Tracer bezeichnet.

Nachteil der bekannten kompetitiven Verfahren ist eine im Vergleich zu Sandwichassays relativ geringe Sensitivität, da anders als bei den immunometrischen Assays die Nachweisreagenzien nicht im Überschuß eingesetzt werden können und somit eine Verschiebung der Gleichgewichtslage zu Gunsten des zu detektierenden Immunkomplexes nicht im gewünschten Maße möglich ist.

Vor der Messung des vom Label ausgesandten Signals ist in der Regel eine Abtrennung des überschüssigen freien Tracerantikörpers (im Falle der Sandwich-Assays und der SPALT-Durchführung) bzw. des ungebundenen Analyttracers (im Falle des klassisch kompetitiven Verfahrens) notwendig.

Bei den als "homogenen Assays" bekannten Verfahren ist eine derartige Trennung nicht erforderlich, da sich die Signale der freien und gebundenen Tracer unterscheiden.

Heterogene Assays besitzen den Nachteil, daß vor der Messung des mit der Analytkonzentration korrelierenden Signals ein oder mehrere Trennschritte erforderlich sind, um den - meist an einer Festphase gebundenen - markierten Immunkomplex von freiem markiertem Reagenz abzutrennen. Im Falle einer manuellen Durchführung ist dies relativ arbeitsaufwendig und erhöht die Fehleranfälligkeit des Verfahrens; auch bei der Durchführung auf einem Analysenautomaten ist der Trennschritt nachteilig, weil für diesen Verfahrensschritt in der Regel eine zusätzliche Baugruppe erforderlich ist.

Es wurden daher schon frühzeitig "homogene Assays" entwickelt. Der homogene Assay, der unter der Bezeichnung EMIT (enzyme-multiplied immunoassay technology) bekannt ist (Biochem. Biophys. Res. Comun. 47: 846, 1972) hat sich für den Nachweis kleiner Moleküle, beispielsweise von Arzneimitteln (z.B. Steroiden), bewährt. In einem modifizierten EMIT, wird die Aktivität des als Label dienenden Enzyms verringert, wenn das Analyt-Enzym-Konjugat an den gegen den Analyten gerichteten Antikörper bindet. Verursacht wird dies anscheinend durch eine verringerte Affinität des Substrats zum aktiven Zentrum des Enzyms in Anwesenheit des Antikörpers, oder durch sterische Behinderung oder durch eine konformative Veränderung des Enzyms.

Eine weitere Variante des EMIT beruht auf der Inhibierung der enzymatischen Aktivität durch das kovalent am Enzym gebundene Analytderivat. Die Aktivität wird in diesem Falle wieder hergestellt, wenn der gegen den Analyten gerichtete Antikörper am Enzym-markierten Analytderivat bindet. Eine andere Variante dieser Methode wurde für größere Analyte, wie beispielsweise IgG entwickelt (Anal. Biochem. 102: 167, 1980). Die mit dieser Methode erzielte Sensitivität ist jedoch recht gering.

Der FETIA (fluorescence excitation transfer immunoassay; J. Biol. Chem. 251: 4172, 1976) beruht auf dem Energietransfer zwischen zwei fluoreszenten Molekülen, wobei das eine mit dem Antikörper und das andere mit dem Analytderivat verknüpft ist. Hier verhindert der nachzuweisende Analyt die Bildung des Komplexes zwischen markiertem Antikörper und markiertem Analytderivat.

Der ECIA (enzyme channelling immunoassay; Anal. Biochem. 1056: 223, 1979; Appl. Biochem. Biotechnol. 6, 53-64, 1981) bedient sich eines Antikörpers und eines Analyttracers, von denen jedes ein unterschiedliches Enzym trägt. Das Produkt der ersten enzymatischen Reaktion stellt das Substrat für die zweite enzymatische Reaktion dar. Die Gesamtgeschwindigkeit beider Reaktionen wird durch diese Co-Immobilisierung deutlich erhöht.

Beim SLFIA (substrate-labeled fluorescent immunoassay) konkurriert ein Analytderivat, das mit einem Enzymsubstrat markiert ist, mit dem Analyten um die Bindungsstellen des Anti-Analyt-Antikörpers. Durch die Bindung des substratmarkierten Analytderivats an den Antikörper kann das Substrat nicht mehr enzymatisch umgesetzt werden (Burd J.F., Feeney J.E., Carrico R.J., Bogulaski R.C.: Clin. Chem. 23, 1402, 1977; Wong R.C., Burd J.F., Carrico R.J., Buckler R.T., Thoma J., Bogulaski R.C.: Clin. Chem. 25, 686, 1979).

Wird eine fluoreszierende Verbindung in Lösung mit polarisiertem Licht angeregt, so ist auch die beobachtete Emission polarisiert. Der Grad dieser Polarisation hängt von der Beweglichkeit des angeregten Moleküls ab. Die durch die Bindung an einen Antikörper abnehmende Beweglichkeit eines fluoreszierenden Tracers dient beim Fluoreszenz-Polarisationsimmunoassay zur Unterscheidung von freiem und gebundenem Tracer.

Beim Fluoreszenz-Protection-lmmunoassay (H. E. Ullmann: Tokai J. Exp. Clin. Med., Vol. 4, Supplement, S. 7 - 32, 1979) handelt es sich um einen homogenen Assay, der nach dem kompetitiven Verfahren arbeitet.
Beim klassisch kompetitiven Assay bleiben im Falle niedriger Analytkonzentrationen genügend Anti-Analyt-Antikörper frei, um den Tracer derart zu binden, daß die Markierung für einen Anti-Fluorescein-Antikörper nicht mehr zugänglich und somit nicht mehr quenchbar wird. Durch Kopplung der Anti-Analyt-Antikörper an eine sterisch anspruchsvolle Komponente kann diese sterische Abschirmung noch effektiver gestaltet werden.

Bei der solid phase antigen Technik verhindert analog die Bindung eines sperrigen Analytderivates am Tracerantikörper dessen gleichzeitige Bindung an den Anti-Fluorescein-Antikörper.
Zur Erhöhung des Quencheffektes wird in einer Variante des Fluoreszenz-Protection-Immunoassays die unspezifische Lichtabsorption von Aktivkohle durch deren Kopplung an den Anti-Fluorescein-Antikörper ausgenutzt (scavenging-Effekt).

Andere Techniken wurden beschrieben, wie beispielsweise der ALFPIA (antigenlabelled fluorescence protection assay; Clin. Chem. 25: 1077, 1979) oder der SPA (scintillation proximity assay; US Patent 4.568.649; WO 90/11524), bei dem dadurch ein Signal erzeugt wird, daß ein radioaktiver Tracer nahe an einen Scintillator bindet.

Die dieser Erfindung zugrunde liegende Aufgabe bestand darin, ein verbessertes Verfahren zur Durchführung eines Immunoassays zur Verfügung zu stellen, das den bekannten Verfahren vor allem in Hinblick auf Sensitivität, Unempfindlichkeit gegenüber potentiell störenden Einflüssen sowie Handhabung überlegen ist.

Die Aufgabe wurde dadurch gelöst, daß neben den üblichen Komponenten eines aus dem Stand der Technik bekannten Immunoassays, wie beispielsweise einem Rezeptor A, der beispielsweise ein Antigen, ein Antigenderivat oder ein Antikörper sein kann und einem Tracer, der abhängig vom gewählten Testaufbau beispielsweise ein markierter Antikörper oder ein markiertes Antigen sein kann, in dem vorliegenden erfindungsgemäßen Verfahren als zusätzliche Komponente ein Rezeptor B eingesetzt wird, der gegen die Markierung gerichtet ist und durch Wechselwirkung mit der Markierung Signal erzeugt oder qualitativ und/oder quantitativ verändert. Dabei wird durch eine geeignete Immobilisierung der Rezeptoren A und B an einer oder mehreren Phasen sichergestellt, daß der Tracer nicht gleichzeitig an die Rezeptoren A und B binden kann. Dies hat den Vorteil, daß ein Tracer, welcher an Rezeptor A gebunden ist und nicht an Rezeptor A gebundener Tracer unmittelbar differenziert werden können, da sie qualitativ und/oder quantitativ unterschiedliche Signale hervorrufen. Damit ist es möglich, Testsysteme zu etablieren, welche gegenüber den aus dem Stand der Technik bekannten eine höhere Sensitivität, verringerte Empfindlichkeit gegenüber potentiellen Störeinflüssen sowie verbesserte Handhabung aufweisen.

Die vorliegenden Erfindung betrifft somit Verfahren zur Bestimmung eines Analyten, worin eine den nachzuweisenden Analyten enthaltende Probe mit einem Rezeptor A und einem eine luminogene Markierung enthaltenden Tracer in Kontakt gebracht werden, so daß der Analyt entweder
a) einen durch die Markierung detektierbaren Komplex mit dem Tracer bildet (immunometrisches Prinzip) oder
b) durch Kompetition mit dem Tracer um die Bindung mit dem gegen den Analyten gerichteten Rezeptor A der Bildung eines Komplexes aus Rezeptor A und Tracer entgegenwirkt (kompetitives Prinzip) oder
c) durch Kompetition mit dem mit dem Analyten strukturidentischen oder dem Analyten strukturähnlichen Rezeptor A der Bildung eines Komplexes aus Rezeptor A und Tracer entgegenwirkt (kompetitives Prinzip),
dadurch gekennzeichnet, daß außerdem
1) ein die luminogene Markierung spezifisch bindender Rezeptor B zugesetzt wird, der durch Wechselwirkung mit der Markierung das entstehende Signal qualitativ und/oder quantitativ verändert, mit Ausnahme eines Signalrepressors, der aus unlöslichen Partikeln besteht, die durch Wechselwirkung mit der Markierung deren Beitrag zur Signalerzeugung unterdrücken, indem sie im relevanten Wellenlängenbereich Licht absorbieren und häufig schwarz sind. und daß
2) das durch die Markierung verursachte Signal bestimmt wird,
wobei durch eine geeignete Immobilisierung der Rezeptoren A und B an oder in einer Phase oder mehreren Phasen sichergestellt wird, daß der Tracer keine Bindung unter gleichzeitiger Beteiligung der Rezeptoren A und B eingehen kann oder nur in einem so geringen Ausmaß, daß Nachweis und Differenzierung unterschiedlicher Analytkonzentrationen dennoch möglich sind.

Im einfachsten Fall handelt es sich bei Rezeptor B um einen Antikörper, der gegen das Label gerichtet ist. Entscheidend ist stets, daß Label und Rezeptor B ein "Paar" bilden, in dem Sinne, daß ihre Wechselwirkung eine Signalerzeugung oder eine Signaländerung zur Folge hat.

Selbstverständlich können auch die Positionen von Label und Rezeptor B vertauscht sein. Beispielsweise wäre dann im Falle eines SPALT-Assays der Anti-Analyt-Antikörper nicht wie üblich mit dem Label markiert, sondern mit dem Rezeptor B konjugiert, während das Label so positioniert ist, daß nur freies (d.h. nicht an dem festphasengebundenen Analyt(derivat) gebundenes) Konjugat aus Anti-Analyt-Antikörper und Rezeptor B eine Bindung mit dem Label eingehen kann.

Unter einem Tracer in seiner allgemeinsten Form wird im Rahmen der vorliegenden Erfindung ein markierter Rezeptor verstanden. Die Markierung (auch "das Label" genannt) kann dabei kovalent oder nicht-kovalent an den Rezeptor gebunden sein. Erfindungsgemäß sind alle aus dem Stand der Technik bekannten Tracer sowie Rezeptoren und Label einsetzbar, sofern sie nur in einer dem Fachmann geläufigen Weise und sinnvoll miteiander kombiniert werden. Der Rezeptorteil des Tracers kann entweder ein einzelner Rezeptor sein oder aus zwei oder mehreren Rezeptoren bestehen, welche untereinander wiederum kovalent oder nicht-kovalent verbunden sein können.

Unter einer Markierung (einem Label) wird eine luminogene Gruppe verstanden.

Im Falle eines klassisch kompetitiven Assays kann bei dem hier beschriebenen Verfahren das Gleichgewicht der analytspezifischen Immunreaktion mit Hilfe einer zugeschalteten Immunreaktion zugunsten der zu detektierenden Immunkomplexe verschoben werden. Dies geschieht im einfachsten Falle dadurch, daß der Analyt einen Analyttracer aus der Bindungsstelle eines Antikörpers A verdrängt und der so "extrahierte" Analyttracer in einer weiteren Immunreaktion von einem Antikörper B gebunden wird.

Während Antikörper A gegen Analyt und Analyttracer gerichtet ist, ist Antikörper B nur in der Lage, den Analyttracer zu binden. Dadurch kann der Analyttracer nicht mehr in üblicher Weise an der Gleichgewichtsreaktion "Antikörper A / Analyt / Analyttracer" teilnehmen.

Durch diese nur gegen den Analyttracer gerichtete Spezifität von Antikörper B und durch Wahl einer geeignet hohen Konzentration des Antikörpers B kann die Gleichgewichtslage sehr günstig beeinflußt werden. Dadurch können die Nachteile einer normalen kompetitiven Immunreaktion bezüglich der Sensitivität verringert werden. Wird durch die Bindung des Tracers an Antikörper B das vom Label ausgesandte Signal verändert, so kann auf eine Abtrennung der zu messenden Komponente verzichtet werden, da in diesem Falle die Signaländerung mit der Konzentration des zu bestimmenden Analyten korreliert. Die Änderung des ausgesandten Signals kann direkt durch die Bindung an den Antikörper B bewirkt werden.

Im Falle eines Sandwich-Assays korreliert die Anzahl der sich bildenden Sandwichkomplexe (bestehend aus Fängerantikörper A, Analyt und Tracerantikörper) mit der Analytkonzentration. Demzufolge korreliert auch die verbleibende Menge an Tracer, die nicht im Sandwichkomplex gebunden ist, mit der Analytkonzentration. Dieser freie Tracer wird von Antikörper B gebunden.

In der vorliegenden Erfindung wird durch eine geeignete Immobilisierung der beiden Rezeptoren A und B sichergestellt, daß Tracer keine stabilen Immunkomplexe eingehen können, an denen Rezeptor A und B gleichzeitig beteiligt sind (beispielsweise Sandwichkomplex aus Antikörper A, Analyttracer und Antikörper B). Eine Möglichkeit der Realisierung ist die Koppelung der Rezeptoren A und B an verschiedene Partikel, wobei Größe und Zusammensetzung der Partikel so gewählt sind, daß eine Bindung des Tracers, an der gleichzeitig Rezeptor A und B beteiligt sind, erschwert oder verhindert wird. Desweiteren können die Rezeptoren A und B auch auf der gleichen Festphase gebunden sein, jedoch in einem Abstand, der groß genug ist, um eine solche Bindung mit einem Tracer zu erschweren oder zu verhindern, an welcher die Rezeptoren A und B gleichzeitig beteiligt sind.

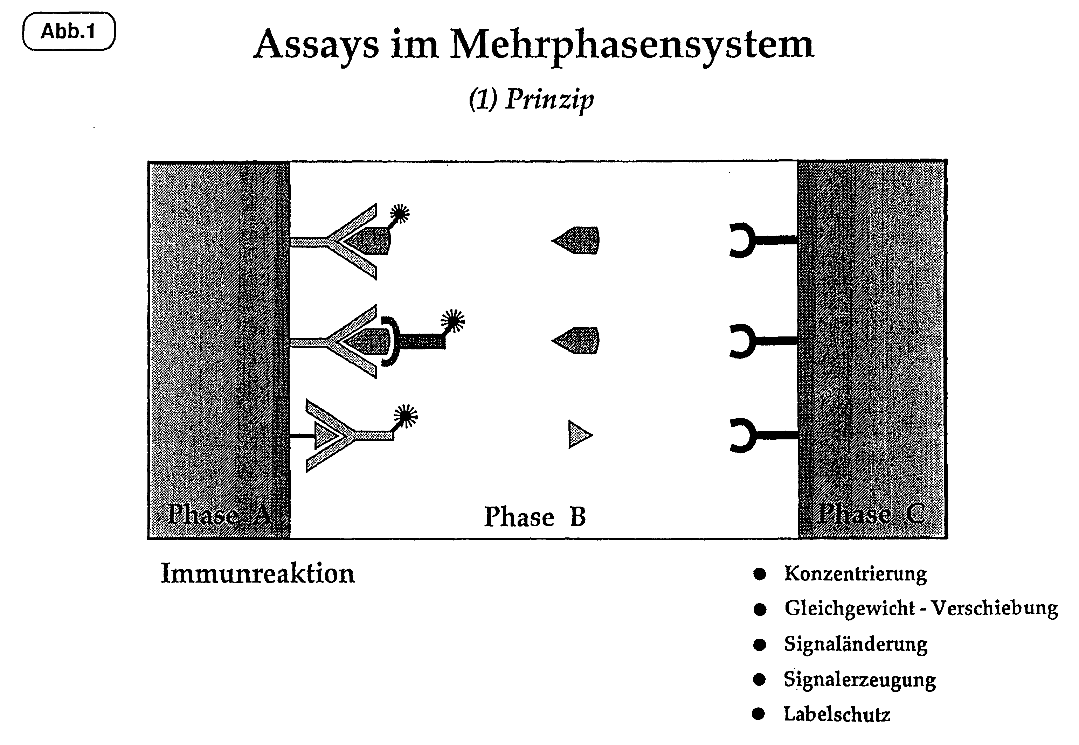

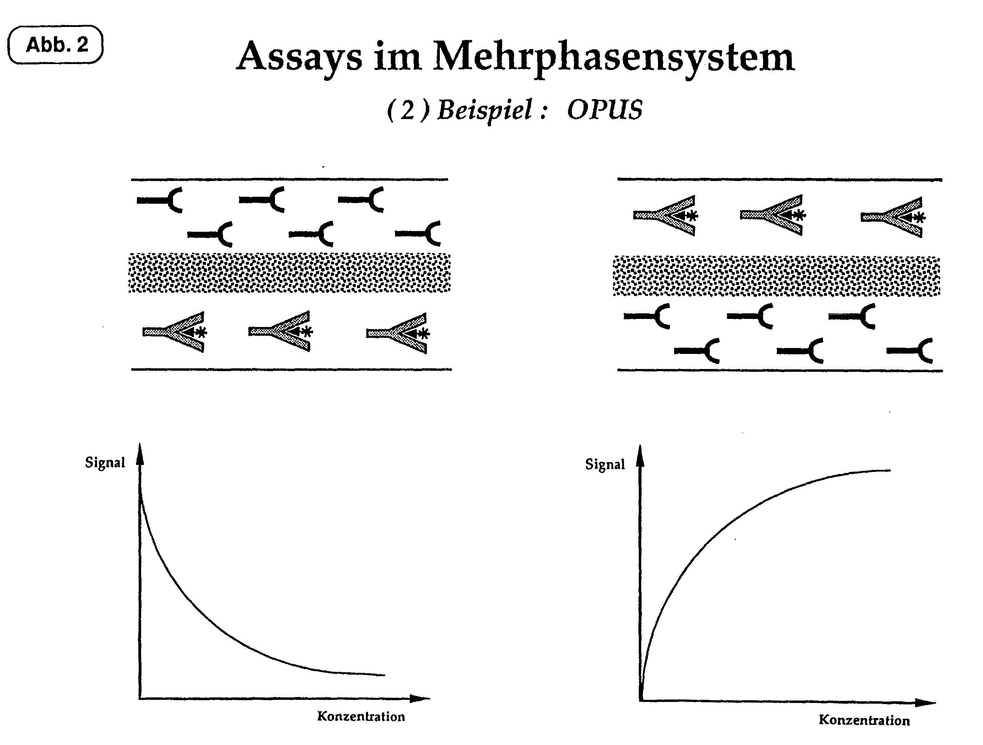

Beispielhaft ist das Prinzip einiger Möglichkeiten schematisch in Abb. 1 dargestellt.

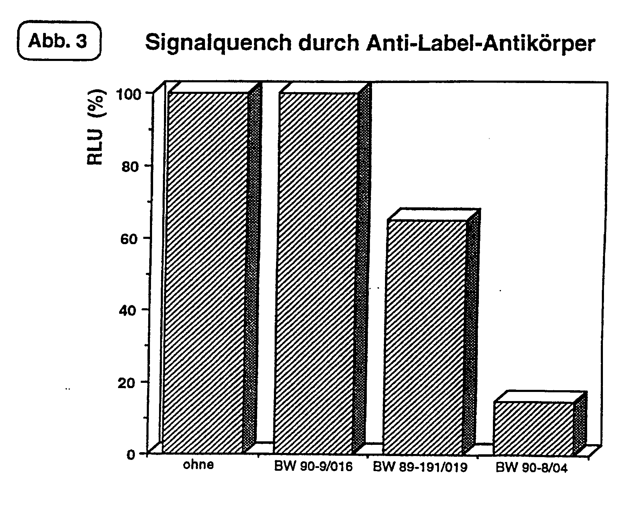

Beispiele derartiger räumlicher Anordnungen sind z.B.:
**1.** Fixierung von Antikörper A und Antikörper B an verschiedene Kunststoffkügelchen, wobei die Größe bzw. die Masse der Kügelchen so groß gewählt wird, daß eine stabile Verbrückung zweier Kügelchen durch eine Bindung, an der Antikörper A und Antikörper B gemeinsam beteiligt sind, nicht möglich ist oder daß aus sterischen Gründen nicht genügend mit Antikörper B beschichtete Kügelchen an ein mit Antikörper A beschichtetes Kügelchen binden können.
**2.** Fixierung von Antikörper A an ein Kunsstoffkügelchen und Fixierung von Antikörper B an die Innenwand des Reaktionsröhrchens.
   Beispiel für eine heterogene Durchführungsvariante:
   In ein mit Antikörper B (=Anti-Label-Antikörper) beschichtetes Polystyrolröhrchen werden mit Antikörper A (Anti-Analyt-Antikörper) beschichtete Kunststoffkügelchen, deren immobilisierte Antikörper A zuvor mit Analyttracer abgesättigt wurden, gegeben. Nach Zugabe der den Analyten enthaltenden Probe und Inkubationspuffer wird die Suspension inkubiert, und anschließend werden die Kunststoffkügelchen entfernt. Das im Röhrchen verbleibende Signal wird gemessen.
**3.** Fixierung von Antikörper A und Antikörper B in verschiedenen Gelphasen eines Testelements, wie es beispielsweise im OPUS®-System (Fa. BDI, Westwood, MA, USA.) eingesetzt wird (Abb. 2).
**4.** Fixierung von Antikörper A und Antikörper B an Membranfasern, wobei durch die Entfernung von Antikörper A und Antikörper B auf einer Faser sichergestellt ist, daß beide Antikörper nicht gleichzeitig eine Bindung mit einem Tracer eingehen können.

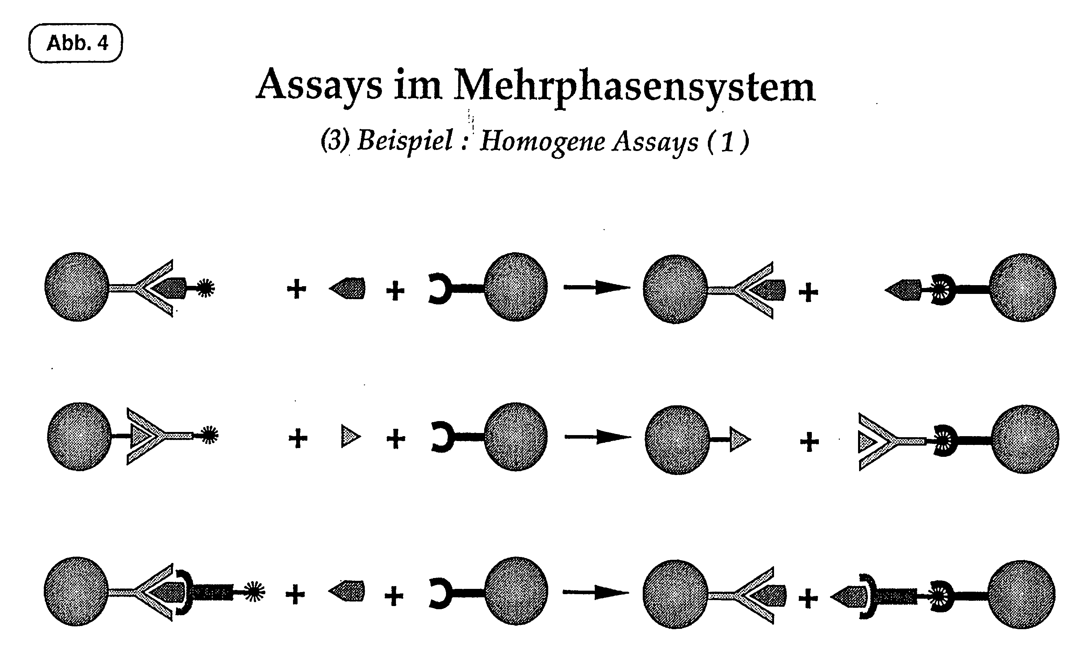

Bewirkt die Bindung des Tracers an den Antikörper B eine Veränderung des Signals, das vom Tracer ausgesandt wird, so kann der Assay in der Weise durchgeführt werden, daß ein Trenn- und/oder Waschschritt überflüssig wird. In diesem Falle korreliert die Signaländerung mit der Konzentration des zu bestimmenden Analyten. Bei der Signaländerung kann es sich um eine qualitative Signaländerung handeln, beispielsweise um eine Verschiebung der Emissionswellenlänge oder/und um eine quantitative Signaländerung, beispielsweise um einen Signalquench. Letzterer ist am Beispiel eines Acridiniumacylsulfonamid-Labels dargestellt in Abb. 3.

Wird das Label/Anti-Label-Antikörper-Paar ersetzt durch ein Enzym-Enzymsubstrat-Paar, so kann an die Stelle einer Signaländerung eine Signalerzeugung treten.

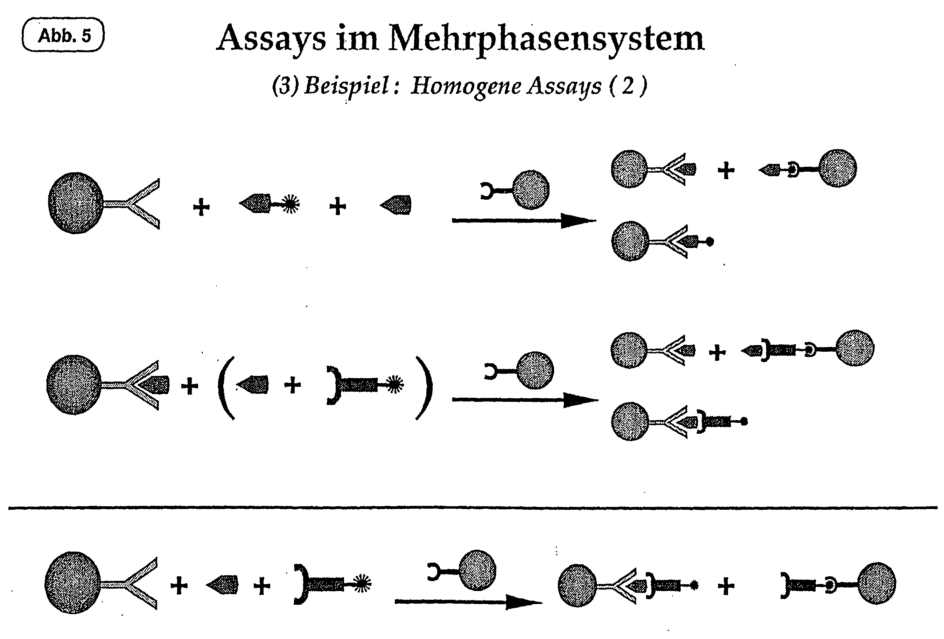

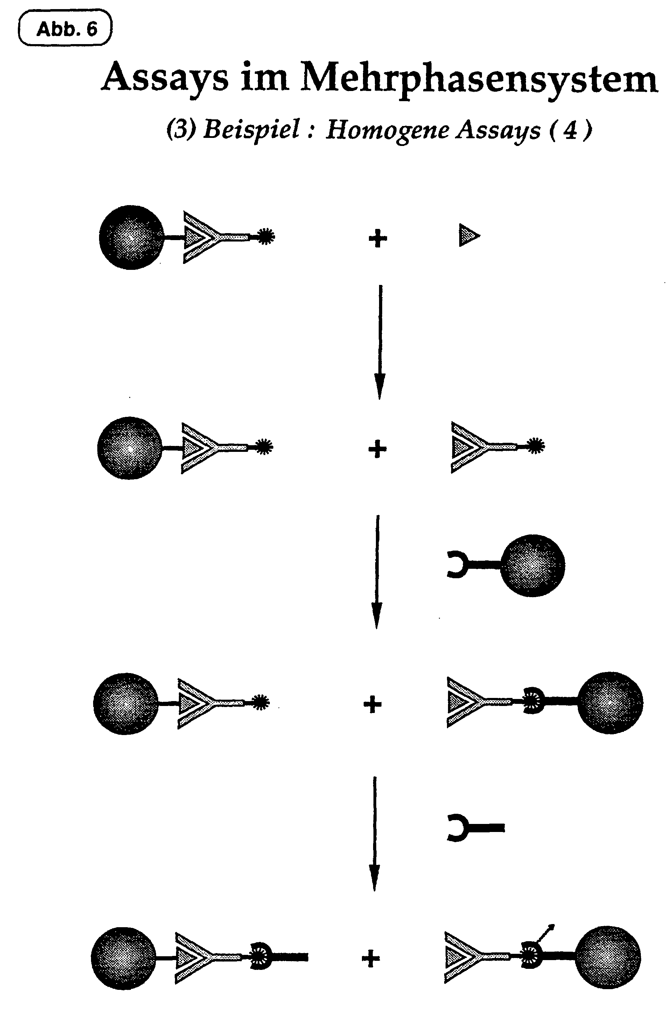

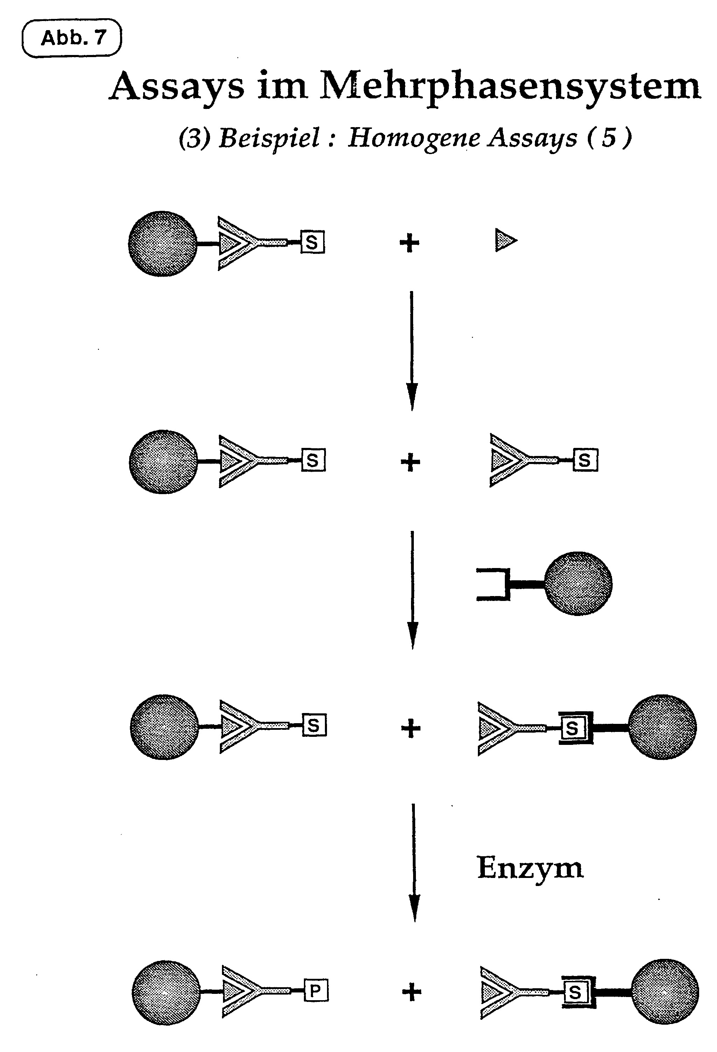

Die Durchführung der Inkubation(sschritte) kann auf verschiedene Weise erfolgen; die Wahl der Reihenfolge ist wie bei der heterogenen Durchführung Teil der normalen Optimierungsarbeit. Beispiele sind:
**1.** Ein präformierter Komplex aus Antikörper A (gebunden an einer Festphase) und Analyttracer wird mit der Analyt enthaltenden Probe inkubiert. Antikörper B (ebenfalls gebunden an einer Festphase) ist während dieser Inkubation von Anfang an anwesend oder wird erst nach Ablauf einer gewissen Zeit t₁ zugegeben. Nach einer Zeit t₂ wird das Signal gemessen (s. obere Zeile der Abb. 4)
**2.** Ein präformierter Komplex aus Analytderivat (gebunden an einer Festphase) und markiertem Antikörper A (SPALT-Prinzip) wird mit der Analyt enthaltenden Probe inkubiert. Antikörper B (ebenfalls gebunden an einer Festphase) ist während dieser Inkubation von Anfang an anwesend oder wird erst nach Ablauf einer gewissen Zeit t₁ zugegeben. Nach einer Zeit t₂ wird das Signal gemessen (s. mittlere Zeile der Abb. 4).
**3.** Ein präformierter Komplex aus Antikörper A (gebunden an einer Festphase), Analyt und Tracerantikörper, also ein präformierter Sandwichkomplex, wird mit der Analyt enthaltenden Probe und mit Antikörper B (ebenfalls gebunden an einer Festphase) inkubiert. Anstelle des Analyten im präformierten Sandwichkomplex kann auch ein modifizierter Analyt eingesetzt werden, um den präformierten Komplex zu instabilisieren und dadurch die Verdrängung des Tracers zu erleichtern (s. untere Zeile der Abb. 4).
**4.** Antikörper A (gebunden an einer Festphase), Analyttracer und Analyt enthaltende Probe werden zusammen inkubiert. Nach Ablauf einer gewissen Zeit t₁ wird Antikörper B (ebenfalls gebunden an einer Festphase) zugegeben und nach Ablauf einer Zeit t₂ das Signal gemessen (s. obere Zeile der Abb. 5)
**5.** Die Analyt enthaltende Probe und Tracerantikörper werden zusammen inkubiert und nach Ablauf einer gewissen Zeit t₁ wird ein präformierter Komplex aus Antikörper A (gebunden an einer Festphase) und Analyt zugegeben. Nach Ablauf einer Zeit t₂ wird Antikörper B (ebenfalls gebunden an einer Festphase) zugesetzt und nach einer Zeit t3 das Signal gemessen (s. mittlere Zeile der Abb. 5).
**6.** Antikörper A (gebunden an einer Festphase), die den Analyten enthaltende Probe und Tracerantikörper werden inkubiert. Nach Ausbildung der Sandwichkomplexe wird Antikörper B (ebenfalls gebunden an einer Festphase) zugegeben und dadurch der nicht im Sandwichkomplex gebundene Traceranteil von Antikörper B gebunden (s. untere Zeile der Abb. 5)
**7.** Ein präformierter Komplex aus Antikörper A (gebunden an einer Festphase) und Analyt oder Analytderivat wird zusammen mit der den Analyten enthaltenden Probe und einem Analyttracer inkubiert. Der nicht vom Antikörper A gebundene Traceranteil wird von Antikörper B gebunden.
   Die Assaydurchführung läßt sich weiterhin variieren, indem zur Immunreaktion zwischen Tracer und Antikörper B eine weitere Immunreaktion zwischen Tracer und einem weiteren Antikörper B' zugeschaltet wird. Während beispielsweise der festphasengebundene Antikörper B die Funktion hat, den nach oder während der analytspezifischen Immunreaktion verbliebenen freien Traceranteil zu binden, um dessen Signal vor einer Änderung zu schützen, kann durch die Zugabe eines nicht festphasengebunden Antikörpers B' das Signal des in der analytspezifischen Bindung involvierten Tracers selektiv verändert werden.
   Dieser zusätzliche Inkubationsschritt kann von Vorteil sein, um beispielsweise bei manueller Durchführung, die Einwirkzeit von Antikörper B genauer steuern zu können oder/und durch entsprechend hohe Konzentrationen an Antikörper B' eine schnellere oder/und deutlicher ausgeprägte Signaländerung zu bewirken, beispielsweise in Fällen, in denen die Menge an anwesendem oder zufügbaren Antikörper B aufgrund seiner Festphasenbindung limitiert ist.
   Im folgenden sind hierzu einige Beispiele aufgeführt:
**8.** Ein präformierter Komplex aus Antikörper A (gebunden an einer Festphase)und Analyttracer wird mit der Analyt enthaltenden Probe inkubiert. Antikörper B (gebunden an einer anderen Festphase) ist während dieser Inkubation von Anfang an anwesend oder wird erst nach Ablauf einer gewissen Zeit t₁ zugegeben. Nach einer Zeit t₂ wird ein Antikörper B' zugegeben und das Signal nach einer geeigneten Zeit t₃ gemessen.
**9.** Ein präformierter Komplex aus Analytderivat (gebunden an einer Festphase) und markiertem Antikörper A (SPALT-Prinzip) wird mit der Analyt enthaltenden Probe inkubiert. Antikörper B (ebenfalls gebunden an einer Festphase) ist während dieser Inkubation von Anfang an anwesend oder wird erst nach Ablauf einer gewissen Zeit t₁ zugegeben. Nach einer Zeit t₂ wird ein Antikörper B' zugegeben und das Signal nach einer weiteren Inkubationszeit t₃ gemessen (s. Abb. 6).
**10.** Ein präformierter Komplex aus Antikörper A (gebunden an einer Festphase), Analyt und Tracerantikörper, also ein präformierter Sandwichkomplex, wird mit der Analyt enthaltenden Probe und mit Antikörper B (ebenfalls gebunden an einer Festphase) inkubiert. Anschließend wird Antikörper B' zugegeben und das Signal gemessen.
**11.** Antikörper A (gebunden an einer Festphase), Analyttracer und Analyt enthaltende Probe werden zusammen inkubiert. Nach Ablauf einer gewissen Zeit t₁ wird Antikörper B (ebenfalls gebunden an einer Festphase) zugegeben und nach Ablauf einer Zeit t₂ Antikörper B' zugesetzt. Anschließend wird das Signal gemessen.
**12.** Die Analyt enthaltende Probe und Tracerantikörper werden zusammen inkubiert und nach Ablauf einer gewissen Zeit t₁ wird ein präformierter Komplex aus Antikörper A (gebunden an einer Festphase) und Analyt zugegeben. Nach Ablauf einer Zeit t₂ wird Antikörper B (ebenfalls gebunden an einer Festphase) zugesetzt und nach einer Zeit t₃ Antikörper B' zugegeben. Anschließend wird das Signal gemessen.
**13.** Antikörper A (gebunden an einer Festphase), die den Analyten enthaltende Probe und Tracerantikörper werden inkubiert. Nach Ausbildung der Sandwichkomplexe wird Antikörper B (ebenfalls gebunden an einer Festphase) zugegeben und dadurch der nicht im Sandwichkomplex gebundene Traceranteil von Antikörper B gebunden. Durch Zusatz von Antikörper B' bindet dieser an den im Sandwichkomplex beteiligten Tracer.
   Alle hier aufgeführten Beispiele lassen sich natürlich auch in einer Variante durchführen, in der die Positionen von Label und Rezeptor B vertauscht sind. Beispiele hierfür sind:
**14.** Ein präformierter Komplex aus Antikörper A (gebunden an einer Festphase) und einem mit Rezeptor B konjugierten Analytderivat wird mit der Analyt enthaltenden Probe inkubiert. Das Label (ebenfalls gebunden an einer Festphase) ist während dieser Inkubation von Anfang an anwesend oder wird erst nach Ablauf einer gewissen Zeit t₁ zugegeben. Nach einer Zeit t₂ wird das Signal gemessen.
**15.** Ein präformierter Komplex aus Analytderivat (gebunden an einer Festphase) und einem mit Antikörper B konjugierten Antikörper A (SPALT-Prinzip) wird mit der Analyt enthaltenden Probe inkubiert. Das Label (ebenfalls gebunden an einer Festphase) ist während dieser Inkubation von Anfang an anwesend oder wird erst nach Ablauf einer gewissen Zeit t₁ zugegeben. Nach einer Zeit t₂ wird das Signal gemessen.
**16.** Ein präformierter Komplex aus Antikörper A (gebunden an einer Festphase), Analyt und mit Antikörper B markierten Anti-Analyt-Antikörper, also ein präformierter Sandwichkomplex, wird mit der Analyt enthaltenden Probe und mit Label (ebenfalls gebunden an einer Festphase) inkubiert.
**17.** Antikörper A (gebunden an einer Festphase), ein mit Antikörper B konjugiertes Analytderivat und Analyt enthaltende Probe werden zusammen inkubiert. Nach Ablauf einer gewissen Zeit t₁ wird Label (ebenfalls gebunden an einer Festphase) zugegeben und nach Ablauf einer Zeit t₂ das Signal gemessen.
**18.** Die Analyt enthaltende Probe und ein mit Antikörper B konjugierte Anti-Analyt-Antikörper werden zusammen inkubiert und nach Ablauf einer gewissen Zeit t₁ wird ein präformierter Komplex aus Antikörper A (gebunden an einer Festphase) und Analyt zugegeben. Nach Ablauf einer Zeit t₂ wird Label (ebenfalls gebunden an einer Festphase) zugesetzt und nach einer Zeit t₃ das Signal gemessen.
**19.** Antikörper A (gebunden an einer Festphase), die den Analyten enthaltende Probe und ein mit Antikörper B konjugierter Anti-Analyt-Antikörper werden inkubiert. Nach Ausbildung der Sandwichkomplexe wird Label (ebenfalls gebunden an einer Festphase) zugegeben und dadurch der nicht im Sandwichkomplex gebundene Anteil an mit Antikörper B konjugierter Anti-Analyt-Antikörper in die Lage versetzt, das Label zu binden.

Die vorliegende Erfindung wird außerdem durch die nachfolgenden Beispiele, welche die Erfindung weiter erläutern aber in keiner Weise einschränken sollen sowie durch die Patentansprüche beschrieben.

### Beispiel 1)

### Lumineszenzimmunoassay (LIA) zur PSA-Bestimmung

### Herstellung der Reagenzien:

### Festphase A:

Magnetpartikel der Fa.Rhone-Poulenc (Artikel-Nr. EM1-100/40) wurden mit einem monoklonalen Anti-PSA-Antikörper (BW 92-283/029; Behringwerke AG, Marburg) nach der Carbodiimid-Methode (G. Wendlberger et al., Synthese von Peptiden, Teil 2, Methoden Org. Chem. (Houben-Weyl) 4th ed. 1952, Bd. XV/2, 1974) beschichtet. Die Beschichtungskonzentration betrug 6 mg Antikörper pro ml 10 %ige Magnetpartikelsuspension. Die gebrauchsfertige Suspension hatte eine Magnetpartikel-Konzentration von 2,5 mg pro ml Lagerpuffer (50 mM 2-Cyclohexylamino-ethansulfonsäure (CHES), 0,5 g NaN₃/l, 3 g Rinderserumrumalbumin/l, pH 8,0).

### Festphase B:

Magnetpartikel wurden wie im Falle der Festphase A mit einem monoklonalen Antikörper. der gegen das Acridinium-N-Acylsulfonamid-Label (EP-A-0 257 541 und EP-A-0 330 050) gerichtet ist (BW 90-9/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) beschichtet.

### Tracer:

Ein monoklonaler Anti-PSA-Antikörper (BW 92-284/03, Behringwerke AG) wurde mit dem Acridinium-N-Acylsulfonamid-Label (EP-A-0 257 541 und EP-A-0 330 050) im molaren Verhältnis 1 + 1 markiert. Die Markierung wurde nach dem in der Literatur beschriebenen NHS-Verfahren (NHS = N-Hydroxysuccinimid-Reaktivgruppe; A.K.Campbell, Chemiluminescence: Principles and Applications in Biology and Medicine, 1. Aufl., VCH/Horwood, Weinheim/Chichester, 1988, S.439.) durchgeführt. Die Reinigung erfolgte mittels Gelpermeationschromatographie (Sephadex G 25). Aufbewahrt wurde der Tracer in einer Konzentration von 300 ng/ml Tracerpuffer (10 mM Natriumacetat, 150 mM NaCI, 2 g Rinderserumalbumin/l, 0,1% Mergal K9N, pH 5,0).

### PSA-Standards:

Der Puffer, in dem PSA (Prostata-spezifisches Antigen, Behringwerke AG) gelöst wurde, hatte folgende Zusammensetzung: 50 mM Tris, 150 mM NaCl, 0,05% NaN₃, 0,01% Tween 20, 0,5 g Rinder-IgG/l, 40 g Rinderserumalbumin/l, 8 mg Titriplex V/I, pH 7,6. Die Standardkonzentrationen betrugen 0, 50, 100, 200 und 400 ng/ml.

### Präformierter Komplex :

Der präformierte Komplex aus Anti-PSA-Antikörper (gebunden an Festphase A) und PSA wurde folgendermaßen gebildet:
1 ml der Festphasen A-Suspension und 1 ml PSA-Lösung [20µg/ml (10mM Phosphatpuffer, pH7,3 mit 1 g Rinder-IgG/l, 0,5 g Natriumazid/l)] wurden 30 Minuten bei 37°C inkubiert, 5 X mit 1 ml Puffer gewaschen (magnetische Abtrennung) und auf ein Endvolumen von 1 ml aufgefüllt.

### Assaydurchführung:

10 µl PSA enthaltende Probe wurden mit 10µl Tracer 2 Minuten bei RT inkubiert. Anschließend wurden 10µl des präformierten Komplexes zugegeben und nach weiteren 2 Minuten 10µl von Festphase B. Die Probe wurde anschließend in einem Luminometer (BeriLux® Analyzer) gemessen (Meßzeit 1 s).

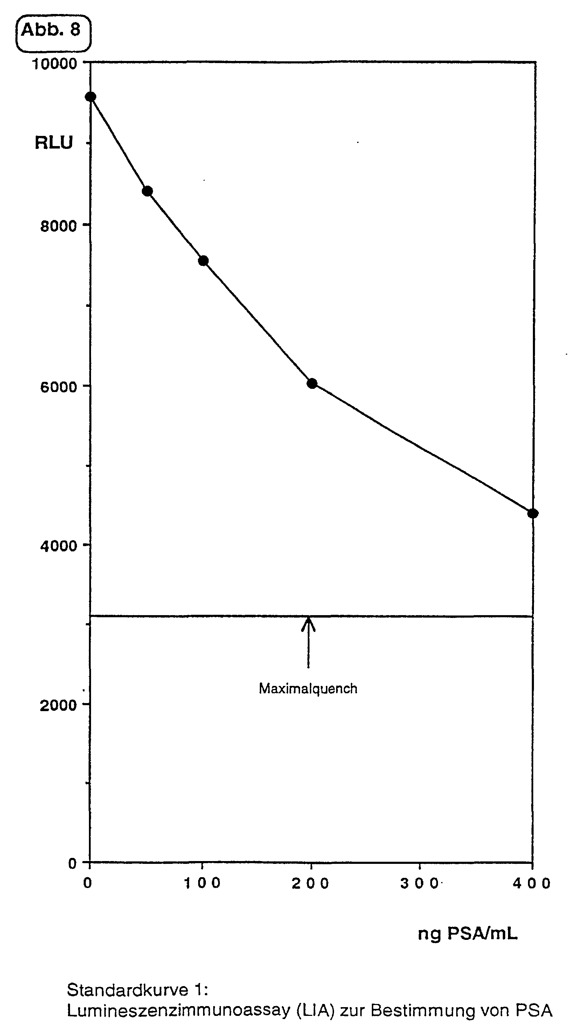

Die Ergebnisse sind in Abb. 8 und die Assaydurchführung ist schematisch in Abb. 8a dargestellt.

### Beispiel 2)

### Immunoluminometrischer Assay (ILMA) zur PSA-Bestimmung

### Herstellung der Reagenzien:

### Festphase A:

Magnetpartikel der Fa.Rhone-Poulenc (Artikel-Nr. EM1-100/40) wurden mit einem monoklonalen Anti-PSA-Antikörper (BW 92-283/029; Behringwerke AG, Marburg) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet.

Die Beschichtungskonzentration betrug 6 mg Antikörper pro ml 10 %ige Magnetpartikelsuspension. Die gebrauchsfertige Suspension hatte eine Magnetpartikel-Konzentration von 10 mg/ml Lagerpuffer (50 mM CHES, 0.5 g NaN₃/l, 3 g Rinderserumalbumin/l, pH 8,0).

### Festphase B:

Magnetpartikel wurden wie im Falle der Festphase A mit einem monoklonalen Antikörper, der gegen das Acridinium-N-Acylsulfonamid-Label (EP-A-0 257 541 und EP-A-0 330 050) gerichtet ist (BW 90-9/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) beschichtet. Die gebrauchsfertige Suspension hatte eine Magnetpartikel-Konzentration von 2,5 mg/ml Lagerpuffer (50 mM CHES, 0,5 g NaN3/l, 3 g Rinderserum-albumin/l, pH 8,0).

### Tracer:

Ein monoklonaler Anti-PSA-Antikörper (BW 92-284/03, Behringwerke AG) wurde mit dem Acridinium-N-Acylsulfonamid-Label (EP-A-0 257 541 und EP-A-0 330 050) im molaren Verhältnis 1 + 0,5 markiert. Die Markierung wurde nach dem in der Literatur beschriebenen NHS-Verfahren (NHS = N-Hydroxysuccinimid-Reaktivgruppe; A.K.Campbell, Chemiluminescence: Principles and Applications in Biology and Medicine, 1. Aufl., VCH/Horwood, Weinheim/Chichester, 1988, S.439.) durchgeführt. Die Reinigung erfolgte mittels Gelpermeationschromatographie (Sephadex G 25). Aufbewahrt wurde der Tracer in einer Konzentration von 30 µg/ml Tracerpuffer (10 mM Natriumacetat, 150 mM NaCI, 2 g Rinderserumalbumin/l, 0,1% Mergal K9N, pH 5,0).

### PSA-Standards:

Der Puffer, in dem PSA (Prostata-spezifisches Antigen. Behringwerke AG) gelöst wurde, hatte folgende Zusammensetzung: 50 mM Tris, 150 mM NaCI, 0,05% NaN₃, 0,01 % Tween 20, 0,5 g Rinder-IgG/l, 40 g Rinderserumalbumin/l, 8 mg Titriplex V/I, pH 7,6. Die Standardkonzentrationen betrugen 0, 0.2, 0.4, 0.8, 1.6, 3.1, 6.2, 12.5 µg/ml.

### Assaydurchführung:

10 µl PSA enthaltende Probe wurden mit 10µl Tracer und 10 µl Festphase A 20 Minuten bei 37°C inkubiert. Anschließend wurden 10µl Festphase B zugegeben und nach weiteren 15 Minuten der Ansatz in einem Luminometer (BeriLux® Analyzer) 1 s gemessen.

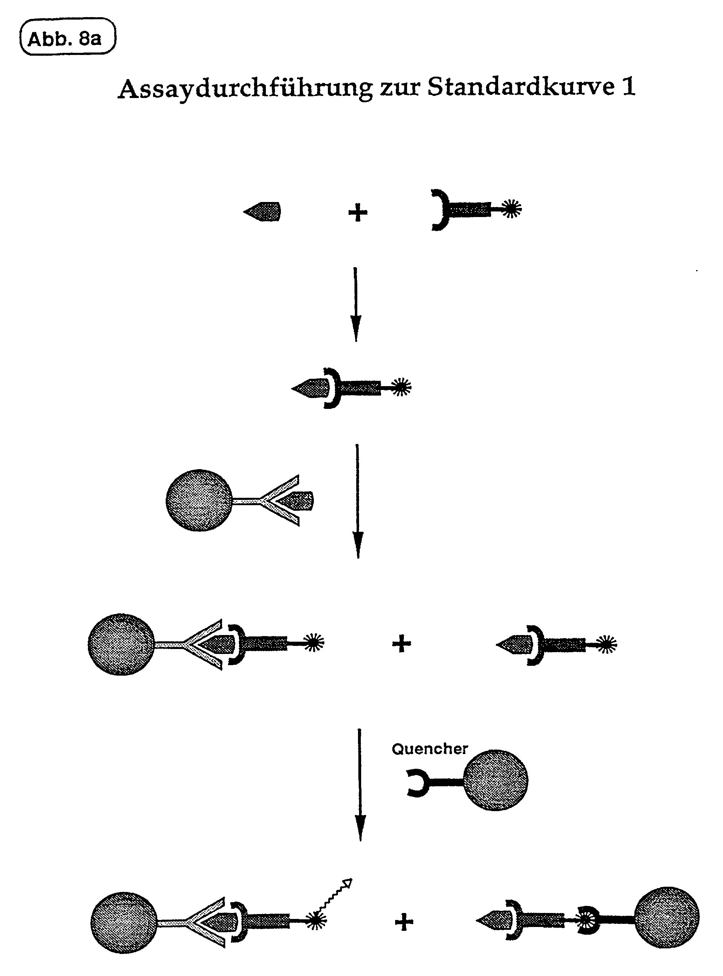

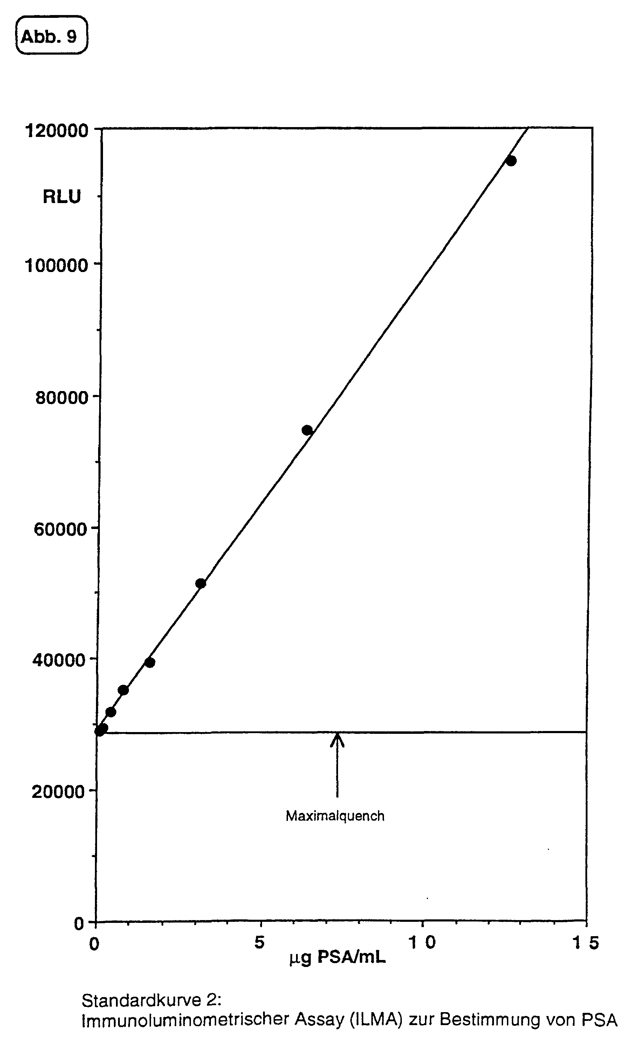

Die Ergebnisse sind in Abb. 9 und die Assaydurchführung ist schematisch in Abb. 9a dargestellt.

### Beispiel 3)

### SPALT-Assay zur Thyroxinbestimmung

### Herstellung der Reagenzien:

### Festphase A:

Magnetpartikel der Fa.Rhone-Poulenc (Artikel-Nr. EM1-100/20) wurden mit dem im BeriLux T3 verwendeten Protein-T3-Konjugat nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 0,3 mg Konjugat pro ml 10 %ige Magnetpartikelsuspension. Die gebrauchsfertige Suspension hatte eine Magnetpartikel-Konzentration von 2,5 mg pro ml Lagerpuffer (50 mM Tris/Citrat-Puffer, 0,5 g NaN₃/l, 3 g Rinderserumalbumin/l, pH 7,0).

### Festphase B:

Magnetpartikel der Fa.Rhone-Poulenc (Artikel-Nr. EM1-100/40) wurden mit einem monoklonalen Antikörper, der gegen das Acridinium-N-Acylsulfonamid-Label (EP-A-0 257 541 und EP-A-0 330 050) gerichtet ist (BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184)nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper pro ml 10 %ige Magnetpartikelsuspension. Die gebrauchsfertige Suspension hatte eine Magnetpartikel-Konzentration von 2,5 mg/ml Lagerpuffer (50 mM CHES, 0,5 g NaN₃/l, 3 g Rinderserumalbumin/l, pH 8,0).

### Tracer:

Ein monoklonaler Anti-T4-Antikörper (BW 86-49/7/1, Behringwerke AG) wurde mit dem Acridinium-N-Acylsulfonamid-Label (EP-A-0 257 541 und EP-A-0 330 050) im molaren Verhältnis 1 + 5 markiert. Die Markierung wurde nach dem in der Literatur beschriebenen NHS-Verfahren (NHS = N-Hydroxysuccinimid-Reaktivgruppe; A.K.Campbell, Chemiluminescence: Principles and Applications in Biology and Medicine, 1. Aufl., VCH/Horwood, Weinheim/Chichester, 1988, S.439.) durchgeführt. Die Reinigung erfolgte mittels Gelpermeationschromatographie (Sephadex G 25). Aufbewahrt wurde der Tracer in einer Konzentration von 40 µg/ml Tracerpuffer (50 mM CitronensäurelPhosphat-Puffer, 2 g Polyethylenglykol 6000/l, 2 g Mowiol/l; pH 6,3).

### T4-Standards:

Der Puffer für die T4-Standards hatte folgende Zusammensetzung: 10 mM Phosphat, 1 g Rinder-IgG/l, 0,5 g NaN₃/l; pH 7,3. Die Standardkonzentrationen betrugen 0, 0.1, 0.5, 1, 5, 10, 50 µg/ml.

### Präformierter Komplex:

Der präformierte Komplex (an Festphase A gebundener Tracer) wurde folgendermaßen hergestellt:
6 ml Festphase A wurden auf 3 ml eingeengt (magnetische Abtrennung der Festphase und Wiederaufnahme in Puffer). Anschließend wurden 0,2 ml Tracer zugegeben und 30 Minuten bei 37°C inkubiert. Mit 10 mM Phosphatpuffer, 1g IgG/l, 0,5 g NaN₃/l, pH 7,3 (Waschpuffer) wurde 5 X gewaschen. Die Lagerung erfolgte in 3 ml Waschpuffer.

### Assaydurchführung:

10 µl T4 enthaltende Probe wurden mit 10µl präformiertem Komplex 2 Minuten bei RT inkubiert. Anschließend wurden 10 µl Festphase B zugegeben und nach weiteren 2 Minuten in einem Luminometer (BeriLux® Analyzer) 1 s gemessen.

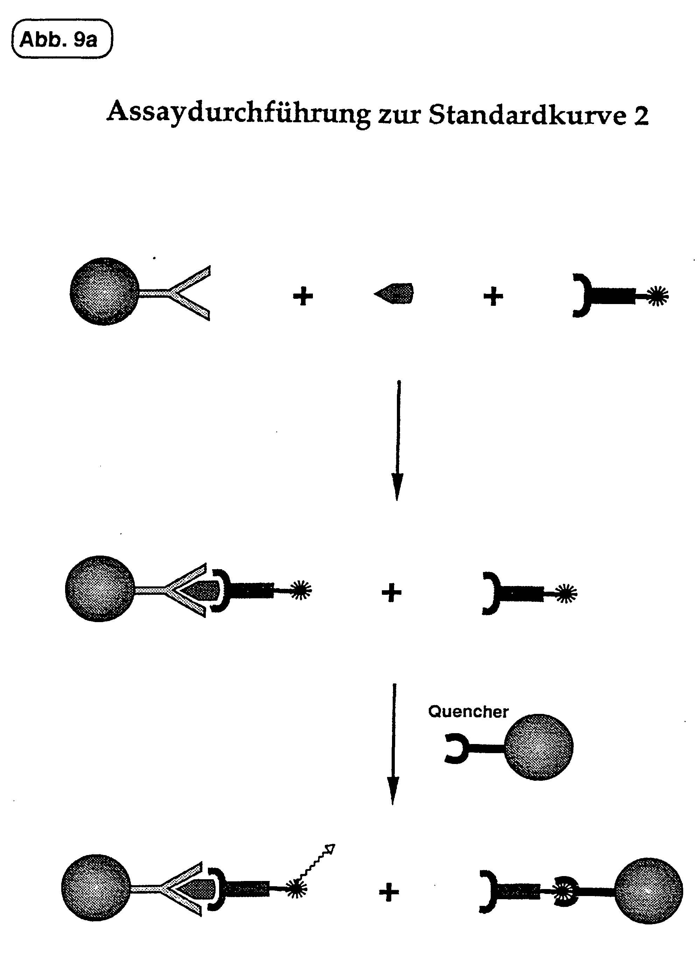

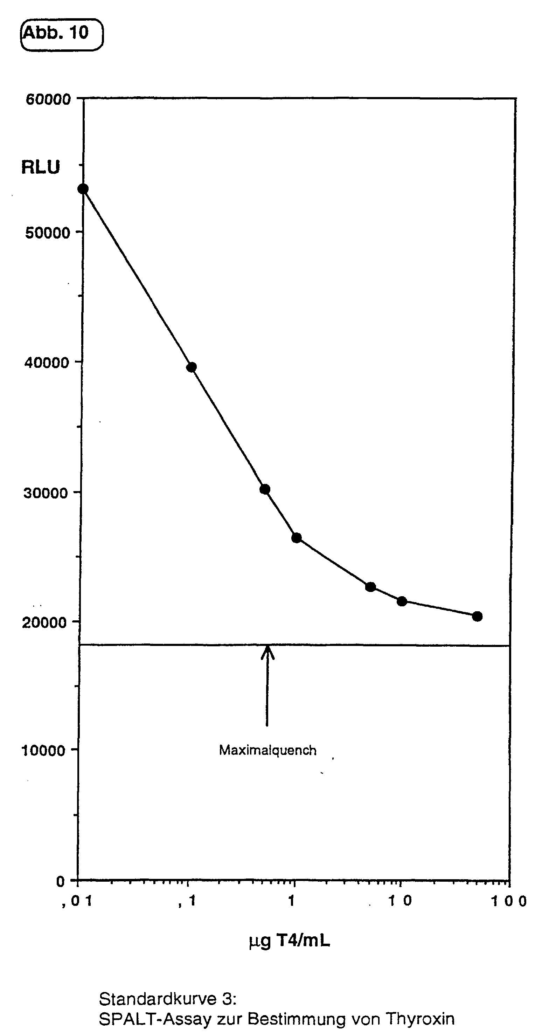

Die Ergebnisse sind in Abb. 10 und die Assaydurchführung ist schematisch in Abb. 10a dargestellt.

### Beispiel 4)

### LIA zur Thyroxinbestimmung (Protector/Quencher-Methode)

### Herstellung der Reagenzien:

### Festphase A:

Magnetpartikel der Fa.Rhone-Poulenc (Artikel-Nr. EM1-100/20) wurden mit einem monoklonalen Anti-T4-Antikörper (BW 86-49/7/1, Behringwerke AG) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 1,25 mg Antikörper/ml 10 %ige Magnetpartikelsuspension. Die gebrauchsfertige Suspension hatte eine Magnetpartikel-Konzentration von 2,5 mg/ ml Lagerpuffer (10,36 g CHES, 0,5 g Natriumazid, 1 g Rinder-IgG/l, pH 8,0).

### Festphase B:

Magnetpartikel der Fa.Rhone-Poulenc (Artikel-Nr. EM1-100/40) wurden mit einem monoklonalen Antikörper, der gegen das Acridinium-N-Acylsulfonamid-Label (EP-A-0 257 541 und EP-A-0 330 050)-Label gerichtet ist (Protector-Antikörper, BW 90-9/016; Behringwerke AG; hinterlegt bei der Deutschen Sammlung von Mikroorganismen und Zellkulturen GmbH, Braunschweig, Deutschland, unter der Hinterlegungsnummer DSM ACC 2183) nach der Carbodiimid-Methode (G. Wendelberger et al., Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper/ml 10 %ige Magnetpartikelsuspension. Die gebrauchsfertige Suspension hatte eine Magnetpartikel-Konzentration von 2,5 mg/ml Lagerpuffer (10,36 g CHES, 0,5 g Natriumazid, 3 g Rinderserumalbumin/l, pH 8,0).

### Tracer:

Der im BeriLux FT3 verwendete Tracer wurde in einer Konzentration von 360 ng/ml Tracerpuffer (10mM Phosphatpuffer, 1g IgG/I, pH 7,3) aufbewahrt.

### Quencher-Antikörper:

Der Anti-Label-Antikörper BW 90-8/04 (Behringwerke AG; hinterlegt bei der Deutschen Sammlung von Mikroorganismen und Zellkulturen GmbH, Braunschweig, Deutschland, unter der Hinterlegungsnummer DSM ACC 2184) wurde in einer Konzentration von 0,1 mg/ml Puffer (10mM Phosphat, 1g IgG/I, 0,5 g Natriumazid / I, pH 7,3) aufbewahrt.

### T4-Standards:

Es wurden die BeriLux® T4 Standards (Behringwerke AG) mit einer Konzentration von 0; 18; 40; 85; 175; 340 ng/ml Serummatrix eingesetzt.

### Präformierter Komplex:

Der präformierte Komplex (an Festphase A gebundener Tracer) wurde folgendermaßen hergestellt:
Zu 2 ml Festphase A wurden wurden 2 ml Tracer gegeben und 30 Minuten bei 37°C inkubiert. Mit 10 mM Phosphatpuffer, 1g IgG/I, 0,5 g Natriumazid/l, pH 7,3 wurde 5 X gewaschen und auf 2 ml aufgefüllt. Anschließend wurden 2 mg ANS (8-Anilinonaphthalin-1-sulfonsäure) zugesetzt.

### Assaydurchführung:

10 µl präformierter Komplex und 10 µl T4 enthaltende Probe wurden 15 Minuten inkubiert. Anschließend wurden im Abstand von jeweils 10 Minuten 10 µl Festphase B und 10 µl Quencher-Antikörper zugegeben und die Suspension in einem Luminometer (BeriLux® Analyzer) 1 s gemessen.
Die Inkubationstemperatur betrug stets 37°C.

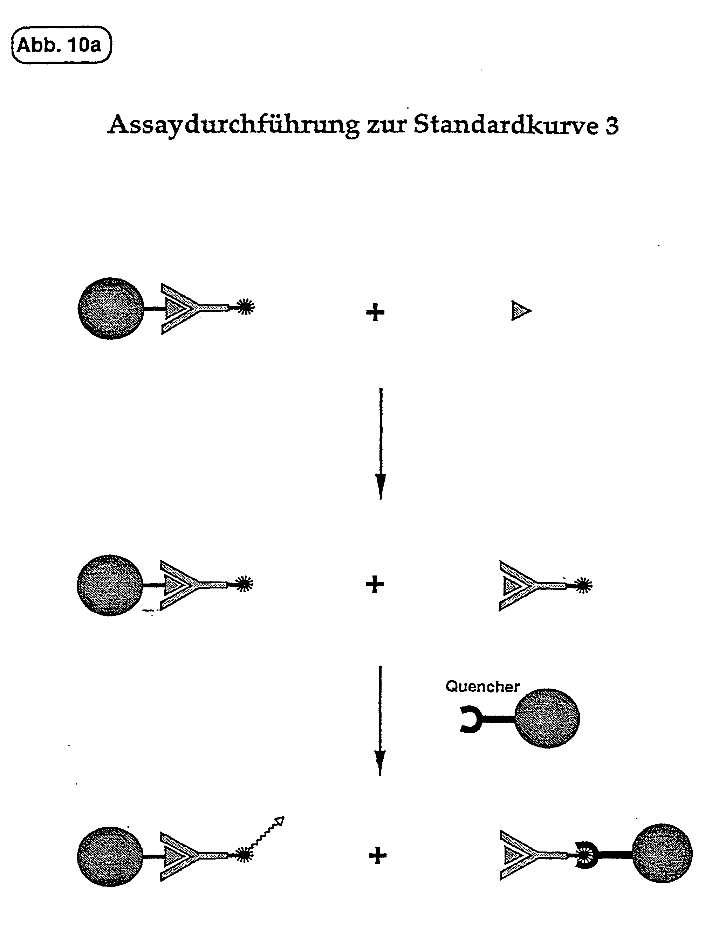

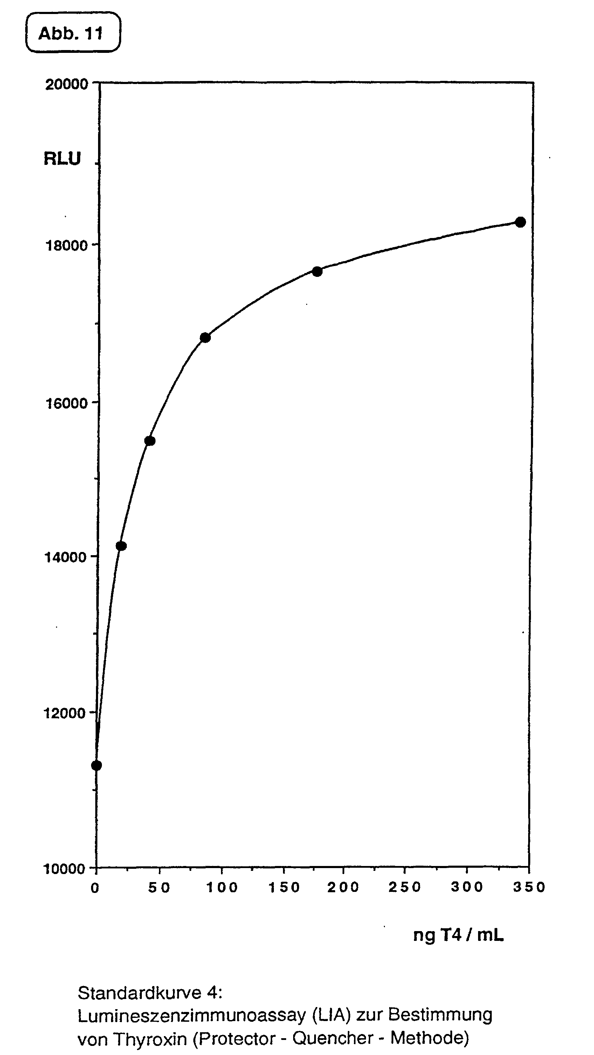

Die Ergebnisse sind in Abb. 11 und die Assaydurchführung ist schematisch in Abb. 11 a dargestellt.

### Beispiel 5)

### LIA zur Thyroxinbestimmung

### Herstellung der Reagenzien:

### Festphase A:

Magnetpartikel der Fa.Rhone-Poulenc (Artikel-Nr. EM1-100/20) wurden mit einem monoklonalen Anti-T4-Antikörper (BW 86-49/7/1, Behringwerke AG) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 1,25 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 1 g Rinder-IgG pro Liter, pH 8,0).

### Festphase B:

Magnetpartikel der Fa.Rhone-Poulenc (Artikel-Nr. EM1-100/40) wurden mit einem monoklonalen Antikörper, der gegen das BeriLux-Label gerichtet ist (Quencher-Antikörper BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184)) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 3 g Rinderserumalbumin pro Liter, pH 8,0).

### Tracer:

Der im BeriLux FT3 verwendete Tracer wurde in einer Konzentration von 360 ng/mL Tracerpuffer (10mM Phosphatpuffer, 1g IgG/L, 0,5 g NaN₃/L, pH 7,3) aufbewahrt.

### T4-Standards:

Es wurden die BeriLux® T4 Standards (Behringwerke AG) mit einer Konzentration von 0; 18; 40; 85; 175; 340 ng/mL Serummatrix eingesetzt.

### Präformierter Komplex:

Der präformierte Komplex (an Festphase A gebundener Tracer) wurde folgendermaßen hergestellt:
Zu 2 mL Festphase A wurden wurden 2 mL Tracer gegeben und 30 Minuten bei 37°C inkubiert. Mit 10 mM Phosphatpuffer, 1g IgG/L, 0,5 g Natriumazid/L, pH 7,3 wurde 5 X gewaschen und auf 2 mL aufgefüllt. Anschließend wurden 2 mg ANS (8-Anilino-naphthalin-1-sulfonsäure) zugesetzt.

### Assaydurchführung:

10 µL präformierter Komplex und 10 µL T4 enthaltende Probe wurden 15 Minuten inkubiert. Anschließend wurde 10 µL Festphase B zugegeben und weitere 10 Minuten inkubiert, bevor die Suspension in einem Luminometer (BeriLux® Analyzer) 1 s gemessen wurde.
Die Inkubationstemperatur betrug stets 37°C.

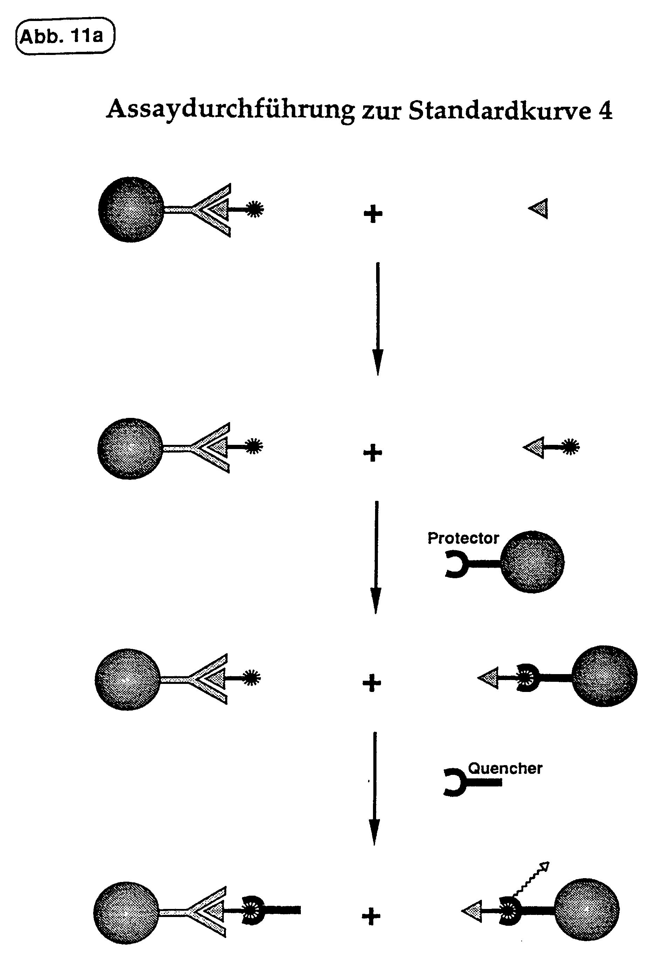

Die Ergebnisse sind in Abb. 12 und die Assaydurchführung ist schematisch in Abb. 12a dargestellt

### Beispiel 6)

### LIA zur FT3-Bestimmung

### Herstellung der Reagenzien:

### Festphase A:

Magnetpartikel der Fa.Rhone-Poulenc (Artikel-Nr. EM1-100/20) wurden mit einem monoklonalen Anti-T3-Antikörper (BeriLux FT3-Antikörper) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 0,25 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 1 g Rinder-IgG pro Liter, pH 8,0).

### Festohase B:

Magnetpartikel der Fa.Rhone-Poulenc (Artikel-Nr. EM1-100/40) wurden mit einem monoklonalen Antikörper, der gegen das BeriLux-Label gerichtet ist (Quencher-Antikörper, BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) nach der Carbodiimid-Methode (G. Wendlberger et al., Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 3 g Rinderserumalbumin pro Liter, pH 8,0).

### Tracer:

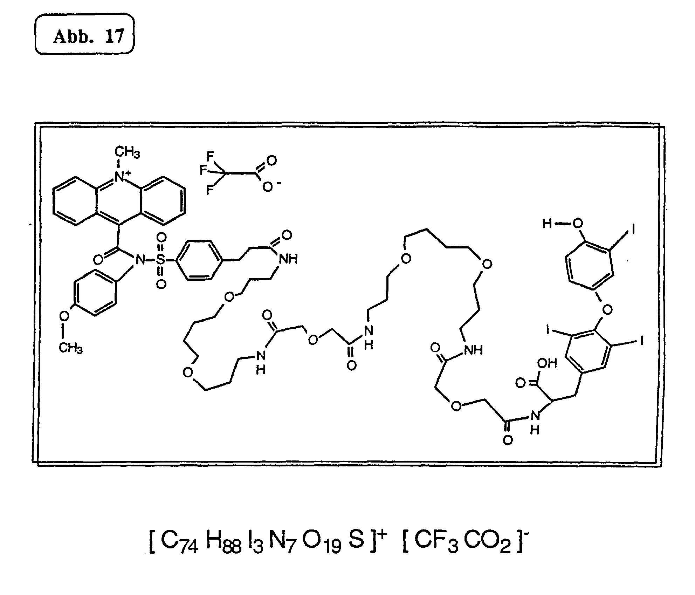

1 ng Haptentracer (T3-Acridiniumtracer; siehe Abb. 17) / mL Tracerpuffer (50 mM Tris/HCl; 150 mM NaCl; 0,5 mg Natriumazid / L; 0,1 g Tween 20 / L; 0,5 g Rinder-IgG / L; 40 g Rinderserumalbumin / L; 8 mg Titriplex V / L; pH 7,6).

### FT3-Standards:

Es wurden FT3-Standards in Serummatrix im Konzentrationsbereich von 0 bis 22 pg/mL eingesetzt.

### Assaydurchführung:

50 µL Probe wurden mit 10 µL Tracer und 10 µL Festphase A 15 Minuten inkubiert. Anschließend wurde 10 µL Festphase B zugegeben und weitere 10 Minuten inkubiert, bevor die Suspension in einem Luminometer (BeriLux® Analyzer) 1 s gemessen wurde. Die Inkubationstemperatur betrug stets 37°C.

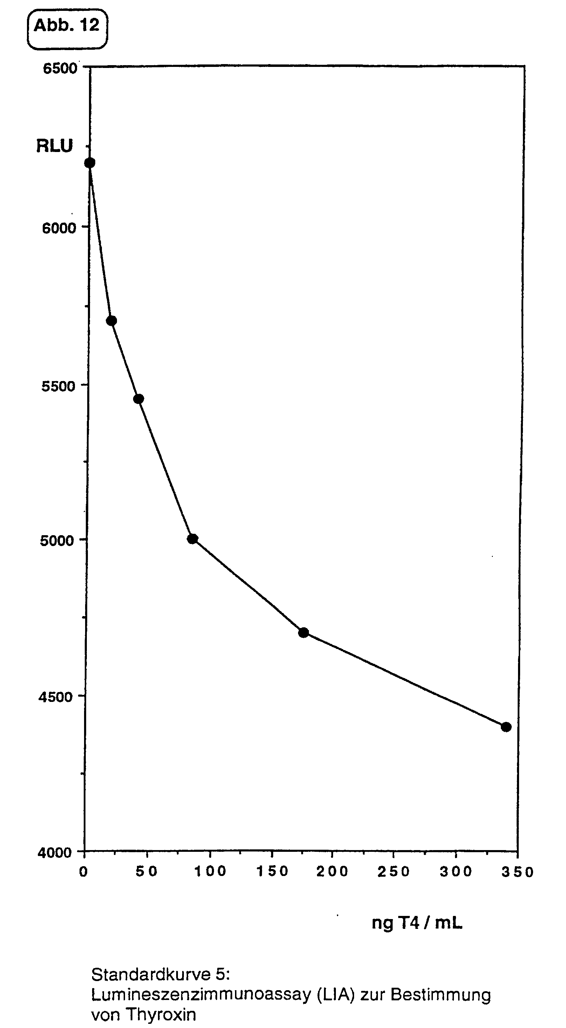

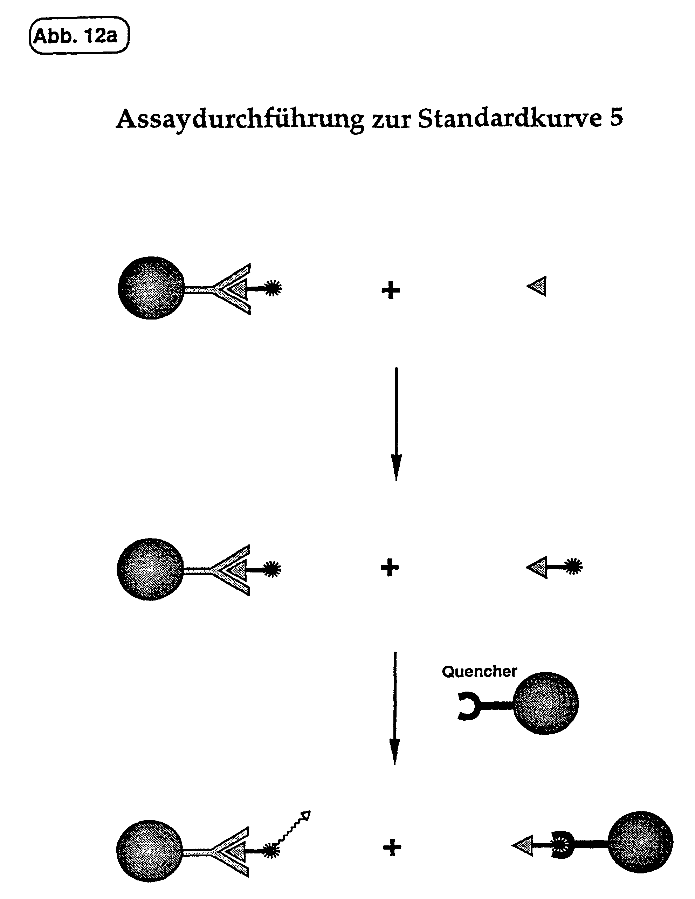

Die Ergebnisse sind in Abb. 13, und die Assaydurchführung ist schematisch in Abb. 13a dargestellt

### Beispiel 7)

### Immunoluminometrischer Assay (ILMA) zur PSA-Bestimmung

### Herstellung der Reagenzien:

### Festphase A:

Magnetpartikel der Fa.Rhone-Poulenc (Artikel-Nr. EM1-100/40) wurden mit einem monoklonalen Anti-PSA-Antikörper (BW 92-283/029; Behringwerke AG, Marburg) nach der Carbodiimid-Methode (G. Wendlberger, P.Stelzel, Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper pro mL 10%ige Magnetpartikelsuspension. Die gebrauchsfertige Suspension hatte eine Magnetpartikel-Konzentration von 10 mg pro mL Lagerpuffer (50 mM CHES, 0,5 g NaN₃ / L, 3 g Rinderserumalbumin / L, pH 8,0).

### Festphase B:

Magnetpartikel wurden wie im Falle der Festphase A mit einem monoklonalen Antikörper, der gegen das BeriLux-Label gerichtet ist (BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) beschichtet. Die gebrauchsfertige Suspension hatte eine Magnetpartikel-Konzentration von 5 mg pro mL Lagerpuffer (50 mM CHES, 0,5 g NaN₃/L, 3 g Rinderserumalbumin / L, pH 8,0).

### Tracerkonzentrat:

Ein monoklonaler Anti-PSA-Antikörper (BW 92-284/03, Behringwerke AG) wurde mit dem BeriLux®-Label im molaren Verhältnis 1 + 10 markiert. Die Markierung wurde nach dem in der Literatur beschriebenen NHS-Verfahren (NHS = N-Hydroxysuccinimid-Reaktivgruppe; A.K.Campbell, Chemiluminescence: Principles and Applications in Biology and Medicine, 1. Aufl., VCH/Horwood, Weinheim/Chichester, 1988, S.439.) durchgeführt. Die Reinigung erfolgte mittels Gelpermeationschromatographie (Sephadex G 25). Aufbewahrt wurde der Tracer in einer Konzentration von 30 µg/mL Tracerpuffer (10 mM Natriumacetat, 150 mM NaCI, 2 g Rinderserumalbumin /L, 0,1% Mergal K9N, pH 5,0).

### Tracer:

Zur Herstellung des gebrauchsfertigen Tracers wird das Tracerkonzentrat mit 0,1 M Phosphatpuffer (pH 6,3 mit 0,15 M NaCI und 1 g Rinderserumalbumin pro Liter) im Verhältnis 1 : 200 verdünnt.

### PSA-Standards:

Der Puffer, in dem PSA (Prostata-spezifisches Antigen, Behringwerke AG) gelöst wurde, hatte folgende Zusammensetzung: 50 mM Tris, 150 mM NaCI, 0,05% NaN₃, 0,01% Tween 20, 0,5 g Rinder-IgG / L, 40 g Rinderserumalbumin / L, 8 mg Titriplex V / L, pH 7,6. Die Standardkonzentrationen betrugen 0, 0.5, 3.2, 10.6, 32, 80, 160 ng/mL.

### Assaydurchführung:

10 µL PSA enthaltende Probe wurden mit 10 µL Festphase A und 20µL Tracer 15 Minuten bei 37°C inkubiert. Anschließend wurden 10µL Festphase B zugegeben und nach weiteren 5 Minuten der Ansatz in einem Luminometer (BeriLux® Analyzer) 0,7 s gemessen. Als Lichtauslösereagenzien wurden 20 mM HNO₃ mit 0,5% H₂O₂ und 125 mM NaOH verwendet.

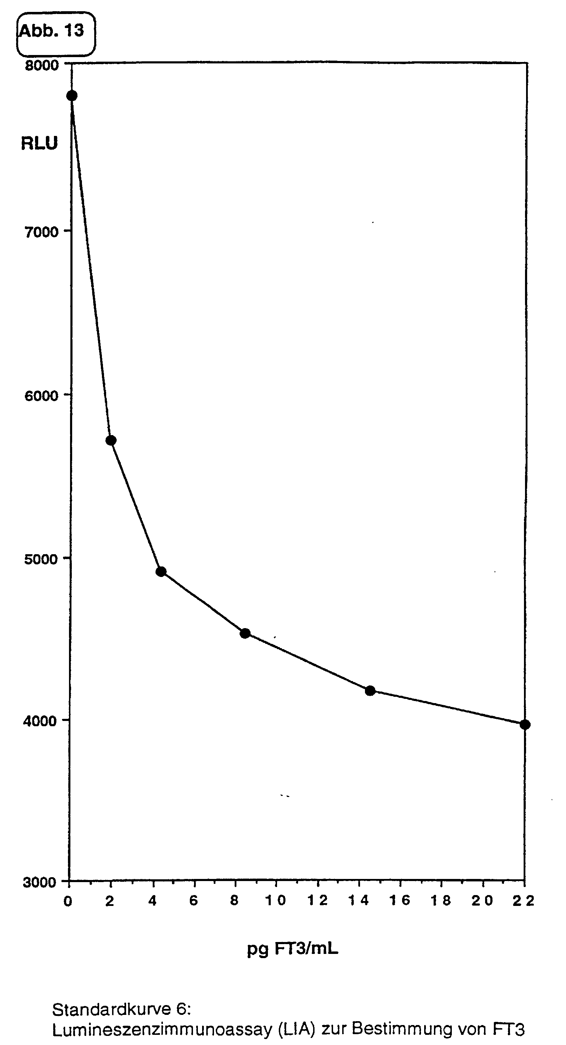

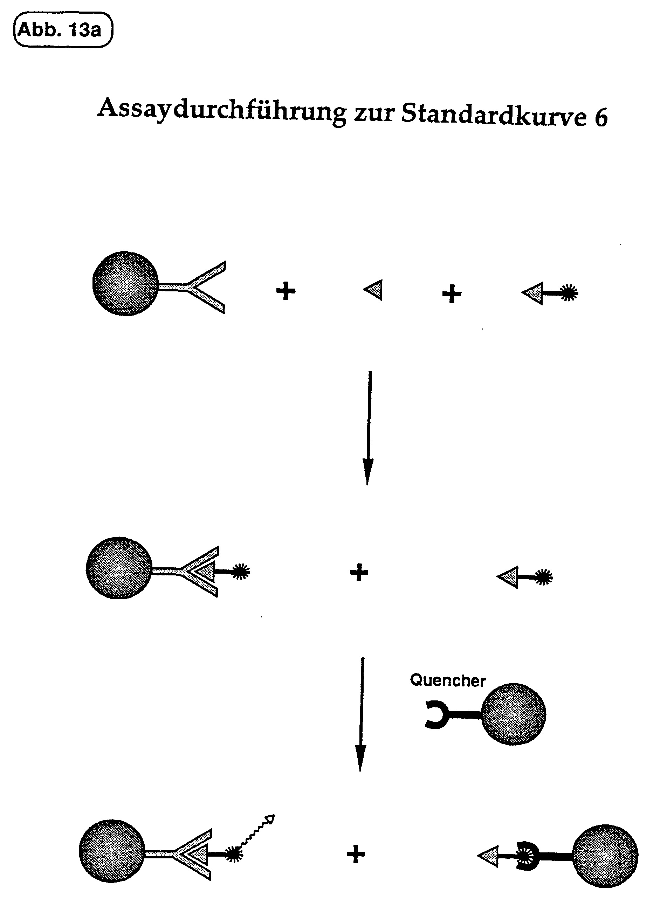

Die Ergebnisse sind in Abb. 14, und die Assaydurchführung ist schematisch in Abb. 9a dargestellt.

### Beispiel 8)

### Immunoluminometrischer Assay (ILMA) zur PSA-Bestimmung

### Herstellung der Reagenzien:

### Festphase A:

Magnetpartikel der Fa.Rhone-Poulenc (Artikel-Nr. EM1-100/40) wurden mit einem monoklonalen Anti-PSA-Antikörper (BW 92-283/029; Behringwerke AG, Marburg) nach der Carbodiimid-Methode (G. Wendlberger, P.Stelzel, Synthese von Peptiden, Teil II, Methoden Org. Chem. (Houben-Weyl) 4th ed. 1952, Bd. XV/2, 1974) beschichtet. Die Beschichtungskonzentration betrug 6 mg Antikörper pro mL 10%ige Magnetpartikelsuspension. Die gebrauchsfertige Suspension hatte eine Magnetpartikel-Konzentration von 10 mg pro mL Lagerpuffer (50 mM CHES, 0,5 g NaN₃ / L, 3 g Rinderserumalbumin / L, pH 8,0).

### Festphase B:

Magnetpartikel wurden wie im Falle der Festphase A mit einem monoklonalen Antikörper, der gegen das BeriLux-Label gerichtet ist (BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) beschichtet. Die gebrauchsfertige Suspension hatte eine Magnetpartikel-Konzentration von 5 mg pro mL Lagerpuffer (50 mM CHES, 0,5 g NaN₃/L, 3 g Rinderserumalbumin /L, pH 8,0).

### Tracerkonzentrat:

Ein monoklonaler Anti-PSA-Antikörper (BW 92-284/03, Behringwerke AG) wurde mit dem BeriLux®-Label im molaren Verhältnis 1 + 10 markiert. Die Markierung wurde nach dem in der Literatur beschriebenen NHS-Verfahren (NHS = N-Hydroxysuccinimid-Reaktivgruppe; A.K.Campbell, Chemiluminescence: Principles and Applications in Biology and Medicine, 1. Aufl., VCH/Horwood, Weinheim/Chichester, 1988, S.439.) durchgeführt. Die Reinigung erfolgte mittels Gelpermeationschromatographie (Sephadex G 25). Aufbewahrt wurde der Tracer in einer Konzentration von 30 µg/mL Tracerpuffer (10 mM Natriumacetat, 150 mM NaCI, 2 g Rinderserumalbumin / L, 0,1% Mergal K9N, pH 5,0).

### Tracer:

Zur Herstellung des gebrauchsfertigen Tracers wird das Tracerkonzentrat mit 0,1 M Phosphatpuffer (pH 6,3 mit 0,15 M NaCl und 1 g Rinderserumalbumin pro Liter) im Verhältnis 1 : 200 verdünnt.

### Meßpuffer:

12,1 g Tris-(hydroxymethyl)-aminomethan, 8,8 g Natriumchlorid, 0,1 g Natriumazid und 10 g Tween 20 werden i990 mL dem. Wasser gelöst und mit 25%iger Salzsäure ein pH von 8,0 eingestellt.
Durch den Einsatz des Meßpuffers in Verbindung mit geeigneten Lichtauslösereagentien (siehe Assaydurchführung) lassen sich Matrixeffekte reduzieren.

### PSA-Standards:

Der Puffer, in dem PSA (Prostata-spezifisches Antigen, Behringwerke AG) gelöst wurde, hatte folgende Zusammensetzung: 50 mM Tris, 150 mM NaCl, 0,05% NaN₃, 0,01% Tween 20, 0,5 g Rinder-IgG /L, 40 g Rinderserumalbumin / L, 8 mg Titriplex V/ L, pH 7,6. Die Standardkonzentrationen betrugen 0, 3.2, 10.6, 32, 80, 160 ng / mL.

### Assaydurchführung:

10 µL PSA enthaltende Probe wurden mit 10 µL Festphase A und 20µL Tracer 15 Minuten bei 37°C inkubiert. Anschließend wurden 10µL Festphase B zugegeben und nach weiteren 5 Minuten 200 µL Meßpuffer. Der Ansatz wurde in einem Luminometer (BeriLux® Analyzer) 0,7 s gemessen. Als Lichtauslösereagenzien wurden 20 mM HNO₃ mit 0,5% H₂O₂ und 125 mM NaOH verwendet.

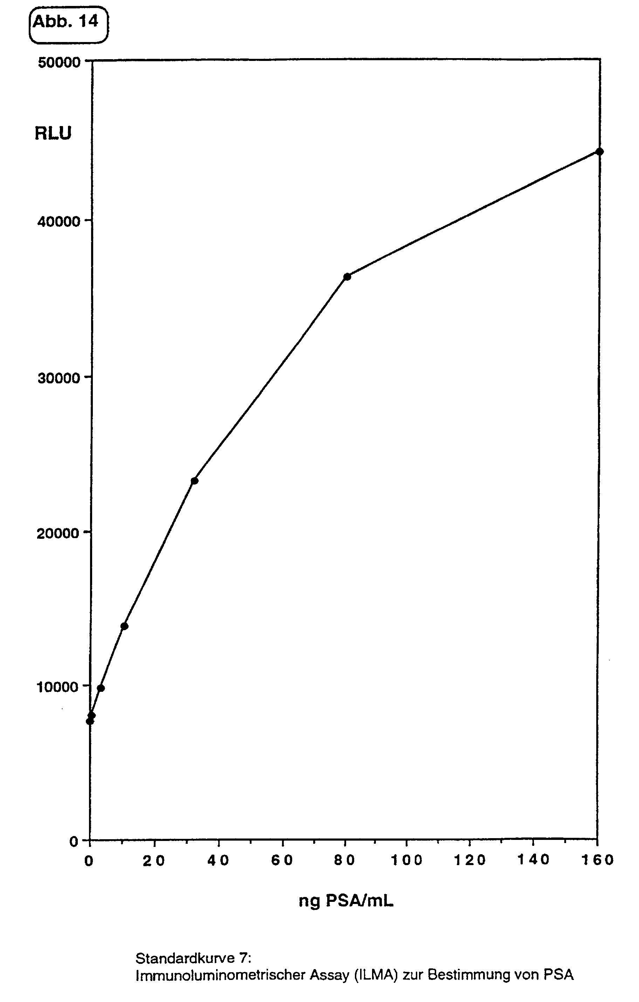

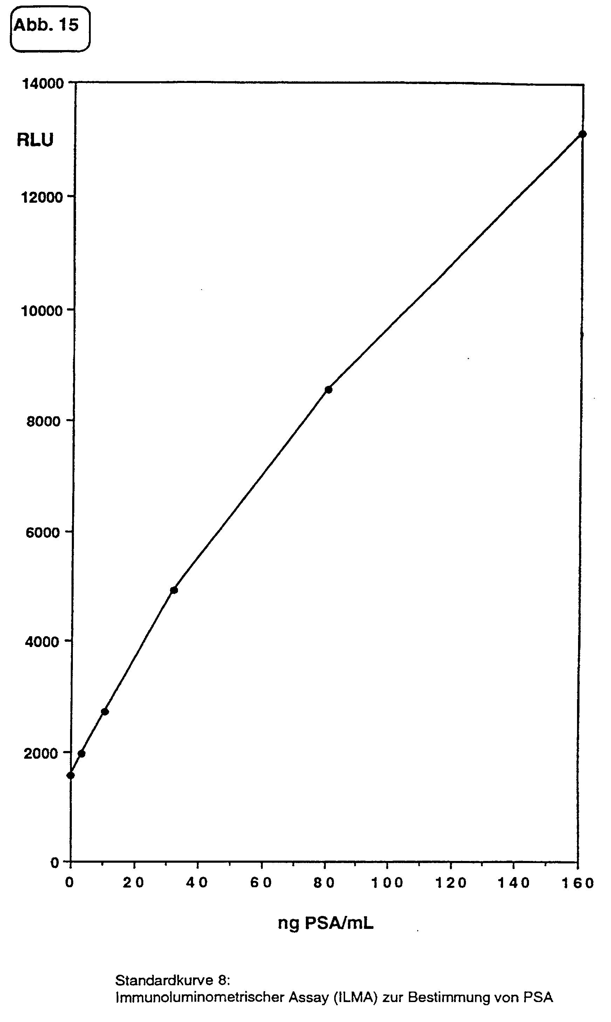

Die Ergebnisse sind in Abb. 15 und die Assaydurchführung ist schematisch in Abb. 9a dargestellt.

### Beispiel 9)

### LIA zur T3-Bestimmung

### Herstellung der Reagenzien:

### Festphase A:

Magnetpartikel der Fa.Rhone-Poulenc (Artikel-Nr. EM1-100/20) wurden mit einem monoklonalen Anti-T3-Antikörper (BeriLux FT3-Antikörper) nach der Carbodiimid-Methode [G.Wendlberger, P.Stelzel, Synthese von Peptiden, Teil II, Methoden Org. Chem. (Houben-Weyl) 4th ed. 1952, Bd. XV/2, 1974] beschichtet. Die Beschichtungskonzentration betrug 0,25 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 3 g Rinderserumalbumin pro Liter, pH 8,0).

### Festphase B:

Magnetpartikel der Fa.Rhone-Poulenc (Artikel-Nr. EM1-100/60) wurden mit einem monoklonalen Antikörper, der gegen das BeriLux-Label gerichtet ist (Quencher-Antikörper. BW 90-8/04; Behringwerke AG; hinterlegt bei der Deutschen Sammlung von Mikroorganismen und Zellkulturen GmbH, Braunschweig, Deutschland, unter der Hinterlegungsnummer DSM ACC 2184) nach der Carbodiimid-Methode [G.Wendlberger, P.Stelzel, Synthese von Peptiden, Teil II, Methoden Org. Chem. (Houben-Weyl) 4th ed. 1952, Bd. XV/2, 1974] beschichtet. Die Beschichtungskonzentration betrug 6 mg Antikörper pro mL 10%ige Magnetpartikelsuspension. Die gebrauchsfertige Suspension hatte eine Magnetpartikel-Konzentration von 2,5 mg pro mL Lagerpuffer (10,36 g CHES, 0,5 g Natriumazid, 3 g Rinderserumalbumin pro Liter, pH 8,0).

### Synthese des Tracers

### Schritt 1: Herstellung eines Streptavidin-T3-Konjugats

a) 3 mg Streptavidin werden in 1.7 mL 0.1 M Na-tetraboratpuffer (mit 10% Dioxan), pH 8.0 gelöst. 28 µL GMBS-Lösung (5 mg g-Maleinimidobutansäure-N-succinimidylester /mL Dioxan) werden zugesetzt. Nach 1 stündiger Reaktion bei RT wird über eine PD10/G25m-Säule in 0.1 M Phosphatpuffer (enthält 5mM EDTA), pH 6.0 umgepuffert. Das resultierende Eluat ("Lösung 1") hat ein Volumen von 2 mL und eine Konzentration von 0.9 mg Streptavidin / mL.
b) 7 mg Triiodthyronin (T3) werden in 700µL DMSO gelöst. Es werden 10 µL N-Ethylmorpholin und 1.6 mg SAMBA (S-Acetylmercaptobemsteinsäureanhydrid), gelöst in 80 µL DMSO, zugegeben. Nach 30 Minuten werden 100 µL 1 M wäßrige Hydroxylaminlösung zupipettiert. Die resultierende Lösung ("Lösung 2") wird 15 Minuten bei RT inkubiert.
c) 1.76 mL Lösung 1 und 41.5 µL Lösung 2 werden gemischt und 1 Stunde bei RT stehengelassen. Anschließend wird über eine PD10/G25m-Säule (Sephadex) in 0.1M K-Phosphatpuffer, pH 7.2, umgepuffert ("Lösung 3"). Nach dem Umpuffern beträgt die Konzentration an Streptavidin-T3-Konjugat 0.6 mg/mL.

### Schritt 2: Markierung des Streptavidin-T3-Konjugats mit BeriLux-Label

a) Synthese des BeriLux-Labels mit Biotin-Gruppe:
   Man legt 200 mg (0.26 mmol) BeriLux-Label (mit NHS-Reaktivgruppe) in 30 mL Acetonitril vor und tropft bei RT eine Lösung von 98 mg (0.26 mmol) N-Biotin-1,8-diamino-3,6-dioxaoctan (Boehringer Mannheim) und 46 µL (0.3 mmol) Triethylamin in 5 mL Acetonitril zu und rührt die Mischung 12 h bei RT, wobei sich die Reaktionsmischung grün färbt. Die Mischung wird filtriert und das Lösungsmittel im Vakuum abdestilliert. Der Rückstand (400 mg; grünes Öl) wird durch präparative Mitteldruckchromatographie (System Büchi) an einer Reversed-Phase Säule [Stationäre Phase: LichroPrep C-18 (Fa. Merck); Mobile Phase: Gradient von Acetonitril/Wasser = 33: 67 + 0.1 Vol% Trifluoressigsäure nach Acetonitril/Wasser = 45: 55 + 0.1Vol.% Trifluoressigsäure] gereinigt. Man isoliert nach Abdestillieren des Acetonitrils i. Vak. und Entfernung des Wassers durch Gefriertrocknung 170 mg gelbes Pulver. MS (FAB): [C₄₇H₅₅N₆O₉S₂]⁺ (berechnet: 911.347; gefunden: 911.350) [CF₃CO₂]⁻ (113).
b) 182 µL Lösung 3 (0.1 mg Streptavidin-T3-Konjugat), 718 µL 0.1 M K-Phosphatpuffer, pH 7.2, 14 µL Acetonitril und 86 µL einer Markierungslösung [10 mg BeriLux-Label (mit Biotingruppe) pro mL Acetonitril] werden zusammenpipettiert und 30 Minuten bei RT inkubiert. Der Tracer wird gelchromatographisch über PD10/G25m gereinigt.

### Herstellung der Tracerlösung

6 ng Tracer / mL Tracerpuffer
Tracerpuffer: 100 mM PBS, pH 6,3 mit 0.2% Rinder-IgG 0,1% Mergal und 0.025% ANS.

### T3-Standards:

Es wurden T3-Standards in Serummatrix im Konzentrationsbereich von 0 bis 7,5 ng/mL eingesetzt.

### Meßpuffer:

100 mM Tris-Puffer, pH 8.0 mit 150 mM NaCl, 1% Tween 20 und 0,01% Natriumazid.

### Assaydurchführung:

50 µL Probe wurden mit 100 µL Tracer und 20 µL Festphase A 30 Minuten bei 37°C inkubiert. Anschließend wurden 10 µL Festphase B zugegeben und nach 10 minütiger Inkubation bei RT 200µL Meßpuffer zupipettiert. Die Suspension wurde in einem Luminometer (BeriLux® Analyzer) 1 s gemessen (Auslösereagenzien R1 = 20mM Salpetersäure mit 0,5% Wasserstoffperoxid; R2 = 125 mM Natronlauge).

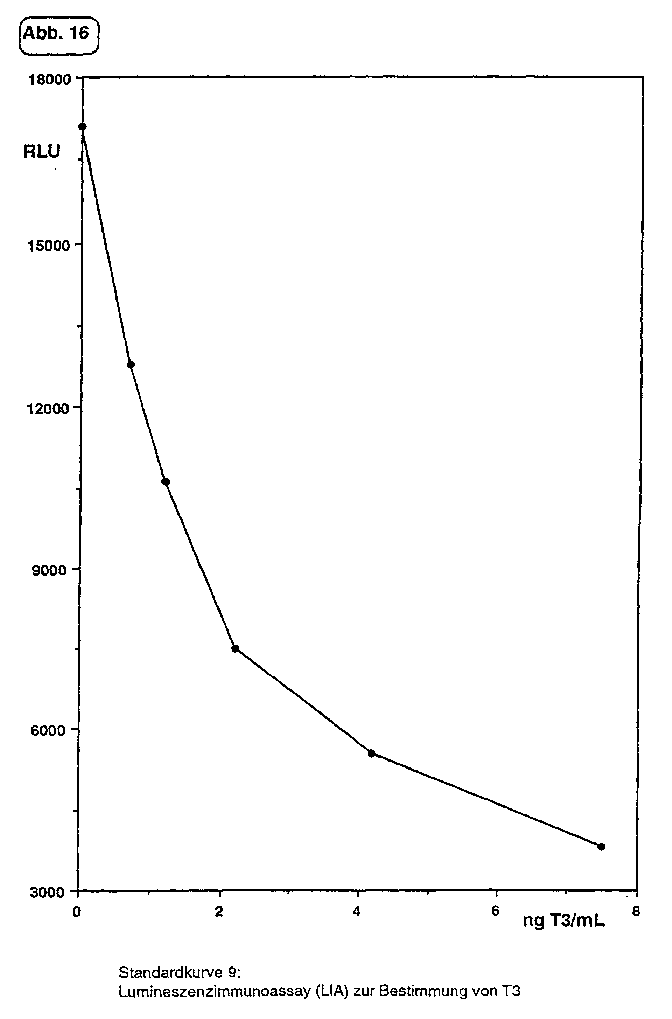

Die Ergebnisse sind in Abb. 16 und die Assaydurchführung ist schematisch in Abb. 13a dargestellt.

### Legende zu Abb. 3

Die Abbildung zeigt die quenchende Wirkung von drei verschiedenen Anti-Label-Antikörpem auf einen mit BeriLux® - Label (Acridiniumacylsulfonamid-Label) markierten Tracerantikörper (Signalaktivität ohne Zusatz eines Anti-Label-Antikörpers = 100 %).
Während sich der Antikörper BW 90-9/016 besonders zum Schutz des Labels vor signaländernden Reagenzien eignet, kann BW 90-8/04 als signaländemder (hier signalquenchender) Antikörper eingesetzt werden. BW 89-191/019 ist für beide Anwendungen weniger gut geeignet.
Die Meßzeit im BeriLux® Analyzer beträgt 1 Sekunde.

## Patentansprüche

1. Verfahren zur Bestimmung eines Analyten, worin eine den nachzuweisenden Analyten enthaltende Probe mit einem Rezeptor A und einem eine luminogene Markierung enthaltenden Tracer in Kontakt gebracht werden, so daß der Analyt entweder
a) einen durch Markierung detektierbaren Komplex mit dem Tracer bildet (immunometrisches Prinzip) oder
b) durch Kompetition mit dem Tracer um die Bindung mit dem gegen den Analyten gerichteten Rezeptor A der Bildung eines Komplexes aus Rezeptor A und Tracer entgegenwirkt (kompetitives Prinzip) oder
c) durch Kompetition mit dem mit dem Analyten strukturidentischen oder dem Analyten strukturähnlichen Rezeptor A der Bildung eines Komplexes aus Rezeptor A und Tracer entgegenwirkt (kompetitives Prinzip),
**dadurch gekennzeichnet, daß** außerdem
1) ein die luminogene Markierung spezifisch bindender Rezeptor B zugesetzt wird, der durch Wechselwirkung mit der Markierung das entstehende Signal qualitativ und/oder quantitativ verändert, mit Ausnahme eines Signalrepressors, der aus unlöslichen Partikeln besteht, die durch Wechselwirkung mit der Markierung deren Beitrag zur Signalerzeugung unterdrücken, indem sie im relevanten Wellenlängenbereich Licht absorbieren und häufig schwarz sind.
und daß
2) das durch die Markierung verursachte Signal bestimmt wird,
wobei durch eine geeignete Immobilisierung der Rezeptoren A und B an oder in einer Phase oder mehreren Phasen sichergestellt wird, daß der Tracer keine Bindung unter gleichzeitiger Beteiligung der Rezeptoren A und B eingehen kann oder nur in einem so geringen Ausmaß, daß Nachweis und Differenzierung unterschiedlicher Analytkonzentrationen dennoch möglich sind.

2. Verfahren gemäß Anspruch 1 nach dem kompetitiven Prinzip, **dadurch gekennzeichnet, daß**
a) der Rezeptor A gegen den Analyten und
b) der Tracer ein Derivat des Analyten ist, gegen das sowohl der Rezeptor A als auch der Rezeptor B gerichtet ist.

3. Verfahren gemäß Anspruch 1 nach dem kompetitiven Prinzip, **dadurch gekennzeichnet, daß**
a) der Tracer ein gegen den Analyten und den Rezeptor A gerichteter Rezeptor ist und
b) Rezeptor A entweder mit dem Analyten strukturidentisch oder ein Derivat des Analyten ist.

4. Verfahren gemäß Anspruch 1 nach dem immunometrischen Prinzip, **dadurch gekennzeichnet, daß**
a) der Rezeptor A gegen den Analyten und
b) der Tracer ein gegen den Analyten gerichteter Rezeptor ist, welcher entweder gegen ein anderes Epitop des Analyten gerichtet ist als Rezeptor A oder, falls der Analyt mehrere gleiche Epitope besitzt, ein anderes oder das gleiche Epitop erkennt wie Rezeptor A.

5. Verfahren gemäß einem oder mehreren der vorhergehenden Ansprüche, **dadurch gekennzeichnet, daß** die Rezeptoren A und B an oder in verschiedenen Phasen gebunden sind.

6. Verfahren gemäß einem oder mehreren der vorhergehenden Ansprüche, **dadurch gekennzeichnet, daß** die Rezeptoren A und B an oder in der gleichen Phase gebunden sind.

7. Verfahren gemäß einem oder mehreren der vorhergehenden Ansprüche, **dadurch gekennzeichnet, daß** mindestens eine Phase eine innere oder äußere Oberfläche eines Feststoffkörpers ist.

8. Verfahren gemäß Anspruch 7, **dadurch gekennzeichnet, daß** der Feststoffkörper eine Membran, ein Röhrchen, eine Mikrotitrationsplatte oder ein Teil davon ist.

9. Verfahren gemäß einem oder mehreren der vorhergehenden Ansprüche, **dadurch gekennzeichnet, daß** mindestens eine Phase aus einer Kunststoffoberfläche besteht.

10. Verfahren gemäß einem oder mehreren der vorhergehenden Ansprüche, **dadurch gekennzeichnet, daß** mindestens eine Phase aus einem oder mehreren metallhaltigen und/oder metallionenhaltigen Kunststoffkügelchen besteht.

11. Verfahren gemäß einem oder mehreren der vorhergehenden Ansprüche, **dadurch gekennzeichnet, daß** mindestens eine Phase aus einem oder mehreren magnetisierbaren Kunststoffkügelchen besteht.

12. Verfahren nach Anspruch 5, **dadurch gekennzeichnet, daß** die beiden Phasen verschiedene Gelschichten sind.

13. Verfahren gemäß einem oder mehreren der vorhergehenden Ansprüche, **dadurch gekennzeichnet, daß** die Markierung eine zur Chemilumineszenz, Biolumineszenz oder Elektrolumineszenz befähigte Gruppe ist.

14. Verfahren gemäß Anspruch 13, **dadurch gekennzeichnet, daß** die Markierung ein Acridiniumester, ein Acridiniumacylsulfonamid, ein Luminol, ein Isoluminol oder ein Derivat dieser Substanzen, ein Dioxetan, ein Luciferin, ein Oxalsäureester oder ein Oxalsäureamid ist.

15. Verfahren gemäß einem oder mehreren der vorhergehenden Ansprüche **dadurch gekennzeichnet, daß** die Markierung direkt an den Tracer gebunden ist.

16. Verfahren gemäß einem oder mehreren der vorhergehenden Ansprüche **dadurch gekennzeichnet, daß** die Markierung indirekt über einen gegen die zu markierende Komponente gerichteten Rezeptor an der zu markierenden Komponente gebunden ist.

17. Verfahren gemäß einem oder mehreren der vorhergehenden Ansprüche **dadurch gekennzeichnet, daß** der Rezeptor B ein monoklonaler Antikörper, ein polyklonaler Antikörper, ein Antikörperfragment, ein chemisch modifizierter Antikörper oder ein chemisch modifiziertes Antikörperfragment ist.

18. Verfahren gemäß einem oder mehreren der vorhergehenden Ansprüche, **dadurch gekennzeichnet, daß** die Markierung eine Acridiniumverbindung ist und der Rezeptor B eine DNA-Doppelhelixstruktur besitzt, deren Funktion darin besteht, die Lichtauslösung des von ihm gebundenen Labels zu verhindern oder zu erschweren.

19. Verfahren gemäß einem oder mehreren der vorhergehenden Ansprüche, **dadurch gekennzeichnet, daß** außerdem ein Rezeptor B' zugesetzt wird, der an die Markierung bindet und dadurch eine qualitativ und/oder quantitativ andere Signaländerung bewirkt als der Rezeptor B.

20. Verfahren nach Anspruch 19, **dadurch gekennzeichnet, daß** der Rezeptor B keine Signaländerung bewirkt.

21. Verfahren gemäß einem oder mehreren der vorhergehenden Ansprüche, **dadurch gekennzeichnet, daß** nach der Inkubation des Rezeptors A, der Probe, des Tracers und des Rezeptors B eine Flüssigkeit zugesetzt wird, um den Probenanteil während der Bestimmung des durch die Markierung verursachten Signals zu verringern oder daß nach der Inkubation von Rezeptor A, Probe und Tracer der Rezeptor B in einem Flüssigkeitsvolumen zugesetzt wird, welches größer ist, als das der eingesetzten Probe.

22. Verfahren gemäß einem oder mehreren der vorhergehenden Ansprüche, **dadurch gekennzeichnet, daß** jeweils anstelle der Markierung der Rezeptor B und anstelle des Rezeptors B die Markierung eingesetzt wird.

23. Verfahren zur Bestimmung eines Analyten, worin eine den nachzuweisenden Analyten enthaltende Probe mit einem präformierten Komplex aus einem eine luminogene Markierung enthaltenden Tracer und einem Rezeptor A in Kontakt gebracht wird, so daß der Analyt entweder
a) den Tracer aus der Bindung mit dem gegen dem Analyten gerichteten Rezeptor A verdrängt oder
b) den mit dem Analyten strukturidentischen oder dem Analyten strukturähnlichen Rezeptor A aus der Bindung mit dem Tracer verdrängt,
**dadurch gekennzeichnet, daß** außerdem
1) ein die luminogene Markierung spezifisch bindender Rezeptor B zugesetzt wird, der durch Wechselwirkung mit der Markierung das entstehende Signal qualitativ und/oder quantitativ verändert, mit Ausnahme eines Signalrepressors, der aus unlöslichen Partikeln besteht, die durch Wechselwirkung mit der Markierung deren Beitrag zur Signalerzeugung unterdrücken, indem sie im relevanten Wellenlängenbereich Licht absorbieren und häufig schwarz sind;
2) und daß nach einem geeigneten Trennschritt das durch den Komplex aus Rezeptor A und Tracer oder Rezeptor B und Tracer hervorgerufene Signal bestimmt wird,
wobei durch eine geeignete Immobilisierung der Rezeptoren A und B an oder in einer oder mehreren Phasen sichergestellt wird, daß der Tracer keinen stabilen Komplex unter gleichzeitiger Beteiligung der Rezeptoren A und B eingehen kann oder nur in einem so geringen Ausmaß, daß Nachweis und Differenzierung unterschiedlicher Analytkonzentrationen dennoch möglich sind.

## Claims

1. A method for determining an analyte, in which method a sample containing the analyte to be detected is brought into contact with a receptor A and a tracer containing a luminogenic label so that the analyte either
a) forms a complex with the tracer, which complex can be detected by the label (immunometric principle), or
b) counteracts the formation of a complex of receptor A and tracer by competing with the tracer for binding to receptor A, which is directed against the analyte (competitive principle), or
c) counteracts the formation of a complex of receptor A and tracer by competing with receptor A, which is structurally identical to the analyte or structurally similar to the analyte (competitive principle),
which comprises additionally
1) adding a receptor B which specifically binds the luminogenic label and which qualitatively and/or quantitatively alters the resulting signal by interacting with the label, with the exception of a signal repressor which is composed of insoluble particles which, by interacting with the label, suppress its contribution to the signal generation by absorbing light in the relevant wavelength range and by frequently being black, and
2) determining the signal brought about by the label,
with a suitable immobilization of receptors A and B on or in one phase or several phases ensuring that the tracer either cannot enter into any binding involving the simultaneous participation of receptors A and B or can enter into such a binding to only such a slight extent that it is nevertheless possible to detect and differentiate differing analyte concentrations.

2. The method as claimed in claim 1 in accordance with the competitive principle, wherein
a) receptor A is directed against the analyte, and
b) the tracer is a derivative of the analyte, against which derivative both receptor A and receptor B are directed.

3. The method as claimed in claim 1 in accordance with the competitive principle, wherein
a) the tracer is a receptor which is directed against the analyte and receptor A, and
b) receptor A is either structurally identical to the analyte or is a derivative of the analyte.

4. The method as claimed in claim 1 in accordance with the immunometric principle, wherein
a) receptor A is directed against the analyte, and
b) the tracer is a receptor which is directed against the analyte, which receptor is either directed against another epitope of the analyte than is receptor A or, if the analyte possesses several identical epitopes, recognizes another epitope or the same epitope as does receptor A.

5. The method as claimed in one or more of the preceding claims, wherein the receptors A and B are bound to or in different phases.

6. The method as claimed in one or more of the preceding claims, wherein the receptors A and B are bound to or in the same phase.

7. The method as claimed in one or more of the preceding claims, wherein at least one phase is an inner or outer surface of a solid body.

8. The method as claimed in claim 7, wherein the solid body is a membrane, a small tube or a microtitration plate or a part thereof.

9. The method as claimed in one or more of the preceding claims, wherein at least one phase comprises a plastic surface.

10. The method as claimed in one or more of the preceding claims, wherein at least one phase comprises one or more metal-containing and/or metal ion-containing plastic beads.

11. The method as claimed in one or more of the preceding claims, wherein at least one phase comprises one or more magnetizable plastic beads.

12. The method as claimed in claim 5, wherein the two phases are different gel layers.

13. The method as claimed in one or more of the preceding claims, wherein the label is a group which is capable of chemiluminescence, bioluminescence or electroluminescence.

14. The method as claimed in claim 13, wherein the label is an acridinium ester, an acridinium acylsulfonamide, a luminol, an isoluminol or a derivative of these substances, a dioxetane, a luciferin, an oxalate or an oxalic amide.

15. The method as claimed in one or more of the preceding claims, wherein the label is bound directly to the tracer.

16. The method as claimed in one or more of the preceding claims, wherein the label is bound indirectly to the component which is to be labeled by way of a receptor which is directed against the component which is to be labeled.

17. The method as claimed in one or more of the preceding claims, wherein the receptor B is a monoclonal antibody, a polyclonal antibody, an antibody fragment, a chemically modified antibody or a chemically modified antibody fragment.

18. The method as claimed in one or more of the preceding claims, wherein the label is an acridinium compound and the receptor B possesses a double helical DNA structure, the function of which comprises preventing, or rendering more difficult, the emission of light from the label which is bound by the receptor.

19. The method as claimed in one or more of the preceding claims, wherein a receptor B' is also added, which receptor binds to the label and thereby brings about a signal change which is qualitatively and/or quantitatively different from that brought about by receptor B.

20. The method as claimed in claim 19, wherein receptor B does not bring about any signal change.

21. The method as claimed in one or more of the preceding claims, wherein, after receptor A, the sample, the tracer and receptor B have been incubated, a liquid is added in order to decrease the sample proportion during the determination of the signal provoked by the label, or wherein, after receptor A, the sample and the tracer have been incubated, receptor B is added in a liquid volume which is greater than that of the sample which is used.

22. The method as claimed in one or more of the preceding claims, wherein, in each case, receptor B is employed in place of the label and the label is employed in place of receptor B.

23. A method for determining an analyte, in which method a sample containing the analyte to be detected is brought into contact with a preformed complex composed of a tracer containing a luminogenic label and a receptor A, so that the analyte either
a) displaces the tracer from the binding with receptor A, which is directed against the analyte, or
b) displaces receptor A, which is structurally identical to the analyte or structurally similar to the analyte, from the binding with the tracer,
which comprises additionally,
1) adding a receptor B which specifically binds the luminogenic label and which qualitatively and/or quantitatively alters the resulting signal by interacting with the label, with the exception of a signal repressor which is composed of insoluble particles which, by interacting with the label, suppress its contribution to the signal generation by absorbing light in the relevant wavelength range and by frequently being black,
2) and, after a suitable separation step, determining the signal which is evoked by the complex composed of receptor A and tracer or receptor B and tracer,
with a suitable immobilization of receptors A and B on or in one phase or several phases ensuring that the tracer either cannot enter into any binding involving the simultaneous participation of receptors A and B or can enter into such a binding to only such a slight extent that it is nevertheless possible to detect and differentiate differing analyte concentrations.

## Revendications

1. Procédé pour la détermination d'un analyte, dans lequel on met un échantillon contenant l'analyte à détecter en contact avec un récepteur A et un traceur contenant un marqueur luminescent, de manière que l'analyte soit
a) forme avec le traceur un complexe détectable par marquage (principe immunométrique), soit
b) s'oppose à la formation d'un complexe de récepteur A et traceur par compétition avec le traceur pour la liaison avec le récepteur A dirigé contre l'analyte (principe compétitif) ou
c) s'oppose à la formation d'un complexe de récepteur A et traceur par compétition avec le récepteur A identique quant à la structure à l'analyte ou ressemblant quant à la structure à l'analyte (principe compétitif),
**caractérisé en ce qu'**en outre
1) on ajoute un récepteur B fixant spécifiquement le marqueur luminescent qui, par interaction avec le marqueur, modifie qualitativement et/ou quantitativement le signal produit, à l'exclusion d'un répresseur de signal qui consiste en particules insolubles qui répriment par interaction avec le marqueur sa contribution à la production de signal par le fait qu'elles absorbent la lumière dans la région de longueur d'onde concernée et sont fréquemment noires,
et **en ce que**
2) on détermine le signal provoqué par le marqueur,
dans lequel on assure par une Immobilisation appropriée des récepteurs A et B sur ou dans une phase ou plusieurs phases, que le marqueur ne peut entrer en aucune liaison avec participation simultanée des récepteurs A et B ou ne peut le faire qu'à un degré tellement faible que la détection et la différenciation de différentes concentrations d'analyte sont néanmoins possibles.

2. Procédé conforme à la revendication 1, selon le principe compétitif, **caractérisé en ce que**
a) le récepteur A est dirigé contre l'analyte et
b) le traceur est un dérivé de l'analyse contre lequel est dirigé aussi bien le récepteur A que le récepteur B.

3. Procédé conforme à la revendication 1, selon le principe compétitif, **caractérisé en ce que**
a) le traceur est un récepteur dirigé contre l'analyte et le récepteur A, et
b) le récepteur A est soit identique quant à la structure à l'analyte ou est un dérivé de l'analyte.

4. Procédé conforme à la revendication 1, selon le principe immunométrique, **caractérisé en ce que**
a) le récepteur A est dirigé contre l'analyte et
b) le traceur est un récepteur dirigé contre l'analyte, qui soit est dirigé contre un autre épitope de l'analyte que le récepteur A, soit, si l'analyte possède plusieurs épitopes identiques, reconnaît le même épitope ou un autre épitope que le récepteur A.

5. Procédé selon une ou plusieurs des revendications précédentes, caractérlsé en ce que les récepteurs A et B sont fixés sur ou dans des phases différentes.

6. Procédé conforme à une ou plusieurs des revendications précédentes, **caractérisé en ce que** les récepteurs A et B sont fixés sur ou dans la même phase.

7. Procédé conforme à une ou plusieurs des revendications précédentes, **caractérisé en ce qu'**au moins une phase est une surface interne ou externe d'un corps solide.

8. Procédé conforme à la revendication 7, **caractérisé en ce que** le corps solide est une membrane, un petit tube, une plaque de microtitrage ou une partie de ceux-ci.

9. Procédé conforme à une ou plusieurs des revendications précédentes, **caractérisé en ce qu'**au moins une phase consiste en une surface de matière plastique.

10. Procédé conforme à une ou plusieurs des revendications précédentes, **caractérisé en ce qu'**au moins une phase consiste en une ou plusieurs sphérules de matière plastique contenant du métal et/ou contenant des ions métalliques.

11. Procédé conforme à une ou plusieurs des revendications précédentes, **caractérisé en ce qu'**au moins une phase consiste en une ou plusieurs sphérules de matière plastique magnétisables.

12. Procédé conforme à la revendication 5, **caractérisé en ce que** les deux phases sont des couches de gel différentes.

13. Procédé conforme à une ou plusieurs des revendications précédentes, **caractérisé en ce que** le marqueur est un groupe apte à la chimioluminescence, la bloluminescence ou l'électroluminescence.

14. Procédé conforme à la revendication 13, **caractérisé en ce que** le marqueur est un ester d'acridinium, un acridiniumacylsulfonamide, un luminol, un isoluminol ou un dérivé de ces substances, un dioxétanne, une luciférine, un ester d'acide oxalique ou un oxalamide.

15. Procédé conforme à une ou plusieurs des revendications précédentes, **caractérisé en ce que** le marqueur est directement lié au traceur.

16. Procédé conforme à une ou plusieurs des revendications précédentes, **caractérisé en ce que** le marqueur est lié indirectement au composant à marquer par l'intermédiaire d'un récepteur dirigé contre le composant à marquer.

17. Procédé conforme à une ou plusieurs des revendications précédentes, **caractérisé en ce que** le récepteur B est un anticorps monoclonal, un anticorps polyclonal, un fragment d'anticorps, un anticorps modifié chimiquement ou un fragment d'anticorps modifié chimiquement.

18. Procédé conforme à une ou plusieurs des revendications précédentes, **caractérisé en ce que** le marqueur est un composé d'acridinium et le récepteur B a une structure de double hélice d'ADN dont la fonction consiste à empêcher ou à rendre difficile l'émission de lumière par l'étiquette liée à celui-ci.

19. Procédé conforme à une ou plusieurs des revendications précédentes, **caractérisé en ce qu'**on ajoute en outre un récepteur B' qui se lie au marqueur et provoque ainsi une qualitativement et/ou quantitativement autre modification de signal que le récepteur B.

20. Procédé conforme à la revendication 19, **caractérisé en ce que** le récepteur B provoque une modification du signal.

21. Procédé conforme à une ou plusieurs des revendications précédentes, **caractérisé en ce qu'**après l'incubation du récepteur A, de l'échantillon, du traceur et du récepteur B, on ajoute un liquide pour diminuer la proportion d'échantillon pendant la détermination du signal provoqué par le marqueur ou **en ce qu'**après l'incubation du récepteur A, de l'échantillon et du traceur on ajoute le récepteur B dans un volume de liquide qui est plus grand que l'échantillon utilisé.

22. Procédé conforme à une ou plusieurs des revendications précédentes, **caractérisé en ce qu'**au lieu du marqueur on utilise le récepteur B et, respectivement, au lieu du récepteur B, on utilise le marqueur.

23. Procédé pour la détermination d'un analyte, dans lequel on met un échantillon contenant l'analyte à détecter en contact avec un complexe préformé d'un traceur contenant un marqueur luminescent et d'un récepteur A, de manière que l'analyte soit
a) déplace le traceur de la liaison avec le récepteur A dirigé contre l'analyte, soit
b) déplace de la liaison avec le traceur le récepteur A identique quant à la structure à l'analyte ou ressemblant quant à la structure à l'analyte,
**caractérisé en ce qu'**en outre
1) on ajoute un récepteur B fixant spécifiquement le marqueur luminescent qui, par Interaction avec le marqueur, modifie qualitativement et/ou quantitativement le signal formé, à l'exclusion d'un répresseur de signal qui consiste en particules insolubles qui, par interaction avec le marqueur, répriment sa contribution à la production de signal par le fait qu'elles absorbent la lumière dans la région de longueur d'onde concernée et sont fréquemment noires ;
2) et **en ce que**, après une étape de séparation convenable, on détermine le signal provoqué par le complexe de récepteur A et traceur ou de récepteur B et traceur,
en assurant par une Immobilisation appropriée des récepteurs A et B sur ou dans une ou plusieurs phases que le traceur ne peut entrer en aucun complexe stable avec participation simultanée des récepteurs A et B ou ne peut le faire qu'à un degré tellement faible que la détection et la différenciation de différentes concentrations d'analyte sont néanmoins possibles.
